# EUROPEAN PATENT SPECIFICATION

(11) **EP 2 686 439 B1**
(45) Date of publication and mention of the grant of the patent: **22.02.2017**
(21) Application number: 12708846.6
(22) Date of filing: 15.03.2012
(51) Int. Cl.: C12Q 1/68

(54) **NOVEL TUMOR MARKER DETERMINATION**
BESTIMMUNG NEUER TUMORMARKER
DÉTECTION À L'AIDE DE NOUVEAUX MARQUEURS TUMORAUX

(30) Priority: 16.03.2011 EP 11158505; 16.03.2011 US 201113049848
(43) Date of publication of application: 22.01.2014
(73) Proprietor: ZEILLINGER, Robert, 2602 Blumau-Neurisshof (AT)
(72) Inventor: ZEILLINGER, Robert, A-2602 Blumau-Neurisshof (AT); OBERMAYR, Eva, A-1200 Wien (AT); CACSIRE CASTILLO TONG, Dan, A-1220 Wien (AT); PILS, Dietmar, A-1180 Wien (AT)
(74) Representative: Redl, Gerda
(86) International application number: PCT/EP2012/054543
(87) International publication number: WO 2012/123536

(56) References cited:
- WO-A2-2006/018290
- OBERMAYR EVA ET AL: "Assessment of a six gene panel for the molecular detection of circulating tumor cells in the blood of female cancer patients.", BMC CANCER, vol. 10, 666, 2010, pages 1-12, XP002640129, ISSN: 1471-2407
- PATEL ILA S ET AL: "Cadherin switching in ovarian cancer progression.", INTERNATIONAL JOURNAL OF CANCER. JOURNAL INTERNATIONAL DU CANCER, vol. 106, no. 2, 20 August 2003 (2003-08-20), pages 172-177, XP002640194, ISSN: 0020-7136

## Description

The present invention relates to a method for determining ovarian cancer in a subject at risk of ovarian cancer.

A tumor marker, also called marker or biomarker, is a substance sometimes found in an increased amount in the blood, other body fluids, or tissues and which may mean that a certain type of cancer is in the body. There are many different tumor markers, each indicative of a particular disease process, and they are used in oncology as a diagnostic, prognostic, or predictive marker or used to monitor cancer therapy.

Often, tumor-specific markers are overexpressed in tumor tissue. Thus, the expression of tumor-specific genes in cancerous tissue is investigated to gain information about prognostic markers and molecular targets for diagnosis or chemical and/or immunological therapy.

Almost two million women in the whole world are diagnosed with breast cancer or other gynecologic malignancies, such as cervical, endometrial, and ovarian cancer each year. Although several advances have been made in early diagnosis during the past few decades, many patients still die of metastasis being the main cause for tumor-related death. In these patients, hematogenous spreading of malignant cells remained undetected at the time of initial diagnosis. This is of particular importance in the case of ovarian cancer with a high metastatic potential.

The use of markers such as estrogen receptor and HER2 for the selection of breast cancer treatment is well established. Recent interest in personalized therapy has led to the search for new markers of prognostic or predictive value.

Tumor cells circulating in the blood of cancer patients, also called circulating tumor cells (CTC) or disseminated tumor cells (DTC), have been described for a series of solid tumor diseases, such as colorectal, lung, kidney, squamous oesophageal, liver, prostate and pancreatic malignancies. Among gynecologic malignancies, most of the research has been done on CTC in breast cancer, whereas only a few data exist on CTC in ovarian, cervical and endometrial cancer. Christofanilli et al. (J Clin Oncol, 2005. 23(7): p. 1420-30) showed that the detection of CTC can predict patient outcome, and the presence of tumor cells in the peripheral blood was considered to be established as an additonal staging parameter. For these reasons, many efforts have been made to develop reliable procedures for the sensitive and specific detection of CTC, either at the protein level, e.g. antibody-based cell staining, or at the mRNA level, e.g. reverse transcription PCR. While the first approach is the gold standard technique for the detection of tumor cells in the bone marrow of breast cancer patients, the latter is supposed to be more sensitive and amenable to high-throughput analysis.

The presence of CTC in a cancer patient undergoing therapy or after therapy would allow the definition of residual disease risk and thereby indicate the potential value of additional or alternative treatment. Minimal residual disease is determined by residual malignant cells; even when so few cancer cells are present that they cannot be found by routine means. Tests for minimal residual disease can detect some early tumors. Persistence of minimal residual disease after primary treatment may be an indication for extensive adjuvant treatment in order to prevent relapse of the disease. Patients, who need intensive and potentially more toxic therapy, can be distinguished from those who do not.

Klein CA (Adv. Cancer Res. 2003; 89:35-67) describe that DTC do not necessarily have the same molecular characteristics as the primary tumors. In contrast, cancer cell evolution obviously explores a multitude of variant cells from which systemic cancer can develop independently. Thus, markers derived from studying the expression profile of tumor tissue would not necessarily be determined in blood samples.

Obermayr et al. (BMC Cancer (2010) Vol. 10, 666) describe a six gene panel for the molecular detection of circulating tumor cells in the blood of female cancer patients. It was found that some ovarian cancer patients had an expression of the CCNE2 and MAL2 markers.

WO2006/018290 A2 discloses some ovarian cancer markers, e.g. TUSC3 and COL3A1.

Patel et al. (International Journal of Cancer (2003) 106(2) 172-177) discloses CDH3 (P-cadherin) as a marker for ovarian cancer, and an increase of P-cadherin mRNA and protein expression levels in ovarian tumor masses with progression to later stages of the disease.

The object of the present invention was to find a new biomarker to evaluate the risk of ovarian cancer in a subject. Specifically it was the object to provide a test determining the relevant marker overexpression in cells of a blood sample.

The object is achieved by the provision of the embodiments of the present invention.

### Summary of the Invention

The present invention refers to a method of determining ovarian cancer disease in a subject, which comprises
- measuring the PPIC expression of cells in a sample of peripheral blood of the subject, and
- comparing to a reference value,
the PPIC overexpression being indicative of a ovarian cancer disease and/or disease progression.

In particular, the invention refers to *ex vivo* methods.

Specifically the sample is obtained from a blood fraction containing mononuclear cells, such as a PBMC fraction. Such a PBMC fraction may be obtained through fractionating blood for the enrichment of blood cells and - amongst others - CTC or epithelial cells, if present. According to a specific embodiment said sample is enriched in circulating tumor cells.

The method according to the invention specifically employs a marker panel comprising PPIC and at least one further marker.

In a preferred embodiment the method according to the invention further comprises
- additionally determining expression of CDH3 in said sample,
wherein the CDH3 expression is indicative of an improved prognosis. This is of particular importance in determining ovarian tumor disease.

According to a specific aspect at least one further marker selected from the group consisting of GPX8, TUSC3, AGR2, COL3A1, LAMB1, MAM (also called SCGB2A2), TFF1, BAIAP2L1, ESRP2 and EpCAM is determined, e.g. in the same sample, optionally employing a set of reagents for the simultaneous or parallel determination of a respective marker panel. If a reduced panel is preferred, the further markers of MAM and EpCAM are less preferred than others or even excluded, because they appear less specific.

Likewise, it turned out that the CCNE2 and MAL2 markers are less indicative for ovarian cancer in a panel specifically used in the method according to the present invention. Therefore, according to a specific embodiment said marker panel does not comprise CCNE2 and/or MAL2.

Specifically the nucleic acid and/or protein expression is determined, either qualitatively or quantitatively. In a preferred embodiment the expression of PPIC and/or at least one further marker is quantitatively determined. In a quantitative determination the significant overexpression of the PPIC and optionally further biomarkers is eventually measured. In a preferred embodiment the method according to the invention employs an internal standard that is measured either side-by-side with the sample determination or used to calibrate the determination system. This is particularly preferred for the quantitative determination method.

The expression is optionally employing amplification methods, among them signal or nucleic acid amplification methods, RT-qPCR, microarrays, immunoassays, such as ELISA, EIA, RIA, western blot, protein arrays, immunocytochemistry or immunohistochemistry methods, as appropriate.

In a preferred method according to the invention, the determination method is highly sensitive, e.g. with a detection limit of a biomarker in a sample containing less than 30 tumor cells/ml blood, preferably less than 15 tumor cells/ml, preferably less than 7 tumor cells/ml or less than one tumor cell/ml, even to a detection limit of one tumor cell per 25ml or less. In a preferred method the determination method is employed in a sample containing at least one tumor cell per 25 ml blood, specifically one tumor cell/ml whole blood.

In a further preferred method, the reference value is determined in a sample of a healthy subject, e.g. from a potentially epithelial cells containing blood fraction of the healthy subject, specifically in a sample containing mononuclear cells, or otherwise derived from a healthy or control subject.

The preferred method according to the invention provides for the comparison of the results of a marker gene expression (determined qualitatively, semi-quantitatively or quantitatively) with a reference value or level. A preferred embodiment comprises a comparative gene expression analysis or the comparison of a gene expression pattern. In a further preferred method, the amount of overexpression differs significantly from the reference value, e.g. at least 1.5 times of the reference value.

The method according to the invention is specifically useful to determine ovarian cancer disease, disease development or disease progression in a patient. This was surprising because prior art methods either referred to biomarkers different from PPIC, or even that PPIC was not indicative of ovarian cancer when testing peripheral blood samples (see Obermayr et al. 2010). Samples are preferably taken from patients who are actually suffering from cancer, in particular who have been diagnosed with cancer, e.g. to monitor the cure of the disease or disease progression.

Yet, according to a specific aspect, the method according to the invention is used for the diagnosis of ovarian cancer, e.g. for diagnosing early stage cancer.

Specifically the subject is suffering from early stage cancer. Thereby a diagnosis, prognosis or prediction is first time possible in such subjects based on the method according to the invention, employing a simple a simple blood test.

According to another aspect of the invention, the subject said subject is undergoing or has received chemotherapy, and the risk of disease progression is determined.

Specifically the subject is suffering from minimal residual disease.

According to another aspect of the invention, the PPIC overexpression is indicative of an increased metastatic potential associated with a shortened survival time, either overall or progression-free survival time.

Thus, the method of the invention is preferably used for diagnosing or aiding in the diagnosis of ovarian cancer and/or to provide indication of or to determine the metastatic potential in an ovarian cancer patient at risk of disease progression. A specific embodiment refers to the use of the method according to the invention for monitoring the disease progression of ovarian cancer in a patient.

According to the invention there is further provided a set of reagents as necessary to determine overexpression of the PPIC biomarker in said sample, e.g. as preferably used for detecting circulating tumor cells in a subject and which further allows the determination of the PPIC expression marker, and optionally further markers, which includes specific protein binders and/or nucleic acids. The set of reagents according to the invention preferably comprises ligands specifically binding the biomarkers, such as antibodies, antibody fragments, or hybridisation probes, which are optionally labelled. The preferred set of reagents also includes an internal standard for the eventual quantitative determination.

### Biomarkers overview

| Gene symbol^{a} | Gene name | UniGene ^{b} accession no. |
|---|---|---|
| *PPIC* | Cyclophilin C, Amino acid and nucleotide sequences (GenBank: S71018) see SEQ ID NO:1 and 2, Figure 3 | Hs.110364 |
| *GPX8* | Glutathione peroxidase 8 (putative) | Hs.289044 |
| *CDH3* | Cadherin-3 | Hs.191842 |
| *TUSC3* | Tumor suppressor candidate 3 | Hs.426324 |
| *AGR2* | Anterior gradient protein 2 homolog | Hs.530009 |
| *COL3A1* | Collagen alpha-1 (III) chain | Hs.443625 |
| *LAMB1* | Laminin subunit beta-1 | Hs.650585 |
| *SCGB2A2 (MAM)* | Mammaglobin A | Hs.46452 |
| *TFF1* | Trefoil factor 1 | Hs.162807 |
| *BAIAP2L1* | Brain-specific angiogenesis inhibitor 1-associated protein 2-like protein 1 | Hs.656063 |
| *ESRP2* | Epithelial splicing regulatory protein 2 | Hs.592053 |
| *EPCAM* | Epithelial cell adhesion molecule | Hs.542050 |

| | | |
|---|---|---|
| ^{a} HUGO nomenclature, where applicable. ^{b} National Center for Biotechnology Information (NCBI), U.S. National Library of Medicine 8600 Rockville Pike, Bethesda MD, 20894 USA | | |

### Figures

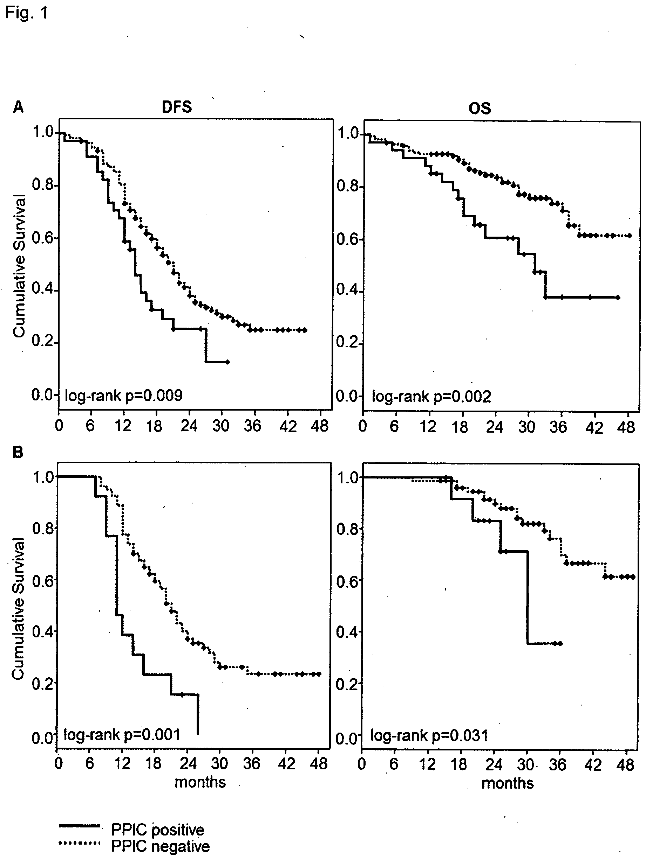

Figure 1: Kaplan-Meier plots of disease free (left panel) and overall survival (right panel) in PPIC negative and PPIC positive EOC patients. Expression levels of 11 markers including the PPIC gene were measured in the CTC fraction of EOC blood samples taken at baseline (A) and during follow-up (B). A sample was defined as CTC-positive, if at least one gene marker was over-expressed beyond the defined threshold. No difference in survival was observed between CTC positive/PPIC negative and CTC negative patients. For this reason, these patients are referred to as PPIC negative patients. Survival outcomes were compared using log-rank testing.

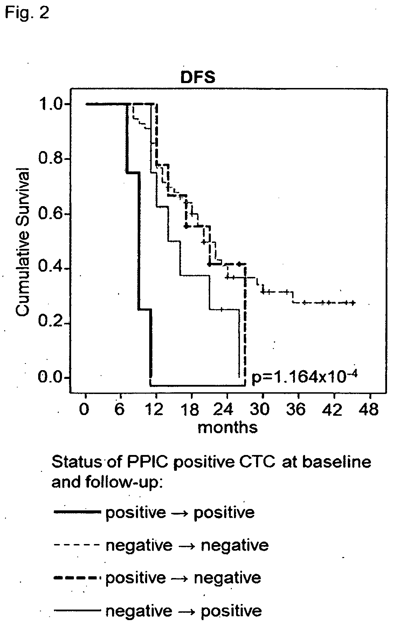

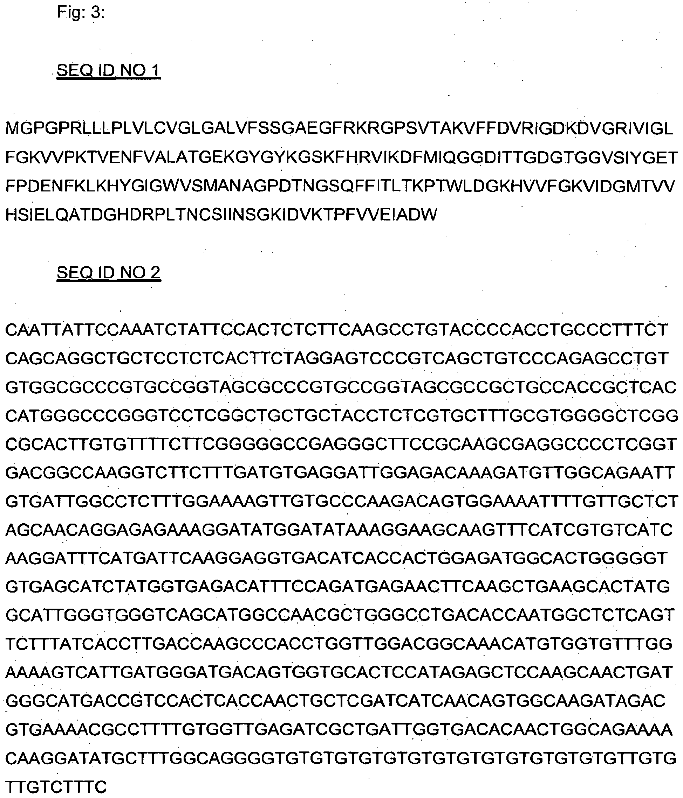

Figure 2: Kaplan-Meier plots of disease free survival stratified by the status of PPIC positive CTC at diagnosis and six months after adjuvant treatment. Blood samples taken both at baseline and follow-up were available from 77 EOC patients.

DFS of patients with negative or positive blood samples at either time points, and with negative→positive or positive→negative blood samples at baseline and follow-up are shown.

### Figure 3:

SEQ ID NO 1: PPIC amino acid sequence (source: NCBI, >sp|P45877|PPIC_HUMAN Peptidyl-prolyl cis-trans isomerase C OS=Homo sapiens GN=PPIC PE=1 SV=1)
SEQ ID NO 2: PPIC nucleotide sequence (source: NCBI, cyclophilin C [human, kidney, mRNA, 883 nt] GenBank: S71018.1)

### Detailed Description

### Peptidyl-prolyl-isomerase C (PPIC)

The protein encoded by this gene located on chromosome 5q23.3 is a member of the peptidyl-prolyl cis-trans isomerase (PPIase) family and is also called cyclophilin C (CypC). Cyps regulate protein folding through PPIase enzymatic and chaperone activities in specific locales of the cells to ensure correct conformation and to counterbalance conformational variations under diverse stress conditions. In addition to PPIase and chaperone activities, each isoform of Cyps has other specific intracellular and extracellular roles. Although roles of Cyps have recently been explored in more details, many physiological and pathological aspects of Cyps' biology still remain unclear [3].

CypC is another Cyp family member that is primarily located in ER, but its role remains to be determined. CypC can form a complex with the COOH-terminal fragment of osteopontin. This complex binds to CD147 to activate Akt1/2 and MMP-2 in 4T07 murine breast cancer cells. This CypC- osteopontin complex regulates in vitro migration and invasion properties of 4T1 and 4T07 breast cancer cells [4].

The PPIC gene or expression product was heretofore not associated with tumor disease or cancer. It was thus surprising that CTC would overexpress PPIC as a diagnostic marker indicating the presence of a gynecologic or ovarian tumor.

### CDH3

The CDH3 gene encoding the cadherin 3, type 1 protein, which is also called P-cadherin is located on the 16q22.1 cytoband. This gene is a classical cadherin from the cadherin superfamily. The encoded protein is a calcium-dependent cell-cell adhesion glycoprotein comprised of five extracellular cadherin repeats, a transmembrane region and a highly conserved cytoplasmic tail. This gene is located in a six-cadherin cluster in a region on the long arm of chromosome 16 that is involved in loss of heterozygosity events in breast and prostate cancer. In addition, aberrant expression of this protein is observed in cervical adenocarcinomas. Mutations in this gene have been associated with congential hypotrichosis with juvenile macular dystrophy.

Cadherins are calcium dependent cell adhesion proteins. They preferentially interact with themselves in a homophilic manner in connecting cells; cadherins may thus contribute to the sorting of heterogeneous cell types. P-cadherin-mediated adherens junctions and the associated signaling pathway play diverse roles in the regulation of tumor cell proliferation, invasiveness and metastatic potential. Upregulation of P-cadherin was frequently observed in various malignancies, including breast, colon, lung and pancreatic tumors, and P-cadherin increase correlated with poor survival of breast cancer patients (Zhang CC, Clin Cancer Res. 2010 16(21):5177-88).

CDH3 was surprisingly found to be an additional prognostic marker according to the invention. Though according to prior art CDH3 overexpression in tumor mass indicated the contrary, CDH3 overexpression of circulating cells actually indicated a decreased risk of developing gynecologic cancer, in particular ovarian cancer, or an improved prognosis.

The term "biomarker" or "biomarker according to the invention" as used herein shall refer to PPIC and optionally CDH3, possibly combined with further biomarkers, such as selected from the panel GPX8, TUSC3, AGR2, COL3A1, LAMB1, TFF1, BAIAP2L1 and ESRP2, optionally further including MAM and/or EpCAM.

The term "PPIC overexpression" as used herein shall refer to samples or cells expressing a higher amount of PPIC, specifically a significantly higher amount, as compared to a reference value, which may be zero or higher, e.g. higher than a threshold or cut-off value, or higher than a reference value derived from a comparable sample.Overexpression may as well be determined by comparison to standards, including internal or external standards.

The term "significantly higher" or ""significant" with respect to the overexpression of a biomarker as used herein shall refer to at least a two-fold higher amount of the standard deviation, preferably at least a three-fold difference. With respect to a specific reference value, such as derived from a standard, training data or threshold, a significant increased amount is understood to refer to an at least 1.5 fold higher amount, preferably at least 2 or 3 fold difference.

The term "ovarian cancer" as used herein shall refer to cancer of the ovaries, e.g. primary of secondary cancer, and specifically including disease conditions in causal relationship with a malignant ovarian tumor.

The term."PBMC fraction" as used herein shall refer to a fraction of peripheral blood including peripheral blood mononuclear cells, either enriched or not, e.g. enriched in white monocytes by density gradient centrifugations. The term shall also include PBMCs preparations isolated from whole blood, e.g. purified PBMC preparations that may be enriched or depleted from CTC that are eventually present in the blood samples.

Thus, the present invention provides for the determination of the PPIC marker alone, or with one or more members of a panel of biomarkers that can be used in a method for determining the disease condition, including detection, diagnosis, in particular early stage diagnosis, prognosis, therapy response prediction, or monitoring solid tumor disease, monitoring therapy, for determining the disease stage, including metastatic disease, and disease status.

In specific aspects of the method of the invention, the method is non-invasive for ovarian cancer diagnosis, which in turn allow for diagnosis of a variety of conditions or diseases associated with solid tumor or cancer disease. In particular, the invention provides a non-invasive non-surgical method for determining the disease condition.

Preferred marker combinations can be derived from the examples below, which are highly specific, e.g. identifying true positive patients, such as a specificity of 50%, 60%, 70% or even more preferred at least 80%. Any marker combination of at least PPIC and optionally one or more markers associated with cancer, which brings about a ratio of positive patients as described above, is considered a preferred combination to determine the risk of gynecologic and specifically ovarian tumor development.

In a specifically preferred method according to the invention, at least one further marker selected from the group of GPX8, TUSC3, AGR2, COL3A1, LAMB1, MAM (also known as SCGB2A2), TFF1, BAIAP2L1, ESRP2 and EpCAM is determined.

### GPX8

The GPX8 gene is located on chromosome 5q11.2 and encodes for the putative glutathione peroxidase 8. This protein belongs to the glutathione peroxidase family and is integrated into the cytoplasmic membrane. The family members 1 to 7 play diverse roles in tumor cells, whereas little is known about the role of GPX8 (Toppo S et al., Antioxid Redox Signal. 2008 Sep;10(9):1501-14. and Brigelius-Flohé R et al., Biochim Biophys Acta. 2009 Nov;1790(11):1555-68).

### TUSC3

This gene is a candidate tumor suppressor gene. It is located on chromosomal band 8p22, where losses of heterozygosity are frequently observed in epithelial tumors including ovarian carcinoma. The gene is expressed in most nonlymphoid human tissues including prostate, lung, liver, and colon. Expression was also detected in many epithelial tumor cell lines. Two transcript variants encoding distinct isoforms have been identified for this gene. Methylation of promotor-associated CpG island leading to the inactivation of tumor suppressor genes was observed in glioblastoma multiforme (Li Q et al, Oncogene 1998 16(24):3197-202).

### AGR2

Human AGR2 is a homolog of the secreted Xenopus laevis protein (XAG-2). In Xenopus, XAG-2 is primarily involved in the induction and differentiation of the cement gland, as well as in the patterning of anterior neural tissues [5]. The human AGR2 gene is located on the chromosomal band 7p21.3. AGR2 gene expression was observed in human tissues rich in epithelial cells. Overexpression occurs in the majority of tumors as compared to matched adjacent benign tissues. Smirnov et al. identified AGR2 as a potential marker for detection of circulating tumor cells in the blood of patients with metastatic cancers [6].

### COL3A1

This gene encodes the pro-alpha1 chains of type III collagen, a fibrillar collagen that is found in extensible connective tissues such as skin, lung, uterus, intestine and the vascular system, frequently in association with type I collagen. Mutations in this gene, which is located on 2q31, are associated with Ehlers-Danlos syndrome types IV, and with aortic and arterial aneurysms. Two transcripts, resulting from the use of alternate polyadenylation signals, have been identified for this gene. Differences in gene expression were observed between advanced and local ovarian carcinoma (up-regulation in advanced stages). The differential gene expression may be related to the carcinogenesis and progression of the malignant growth (Tapper J et al., Cancer Genet Cytogenet, 2001 128(1):1-6).

### LAMB1

Laminins, a family of extracellular matrix glycoproteins, are the major noncollagenous constituent of basement membranes. They have been implicated in a wide variety of biological processes including cell adhesion, differentiation, migration, signalling, neurite outgrowth and metastasis. Laminins are composed of three non-identical chains: laminin alpha, beta and gamma (formerly A, B1, and B2, respectively) and they form a cruciform structure consisting of three short arms, each formed by a different chain, and a long arm composed of all three chains. Each laminin chain is a multidomain protein encoded by a distinct gene. Several isoforms of each chain have been described. Different alpha, beta and gamma chain isomers combine to give rise to different heterotrimeric laminin isoforms, which are designated by Arabic numerals in the order of their discovery, i.e. alpha1beta1gamma1 heterotrimer, is laminin 1. The biological functions of the different chains and trimer molecules are largely unknown, but some of the chains have been shown to differ with respect to their tissue distribution, presumably reflecting diverse functions in vivo. This gene encodes the beta chain isoform laminin, beta 1. The beta 1 chain has seven structurally distinct domains, which it shares with other beta chain isomers. The C-terminal helical region containing domains I and II are separated by domain alpha, domains III and V contain several EGF-like repeats, and domains IV and VI have a globular conformation. Laminin, beta 1 is expressed in most tissues that produce basement membranes, and is one of the three chains constituting laminin 1, the first laminin isolated from Engelbreth-Holm-Swarm (EHS) tumor. A sequence in the beta 1 chain that is involved in cell attachment, chemotaxis, and binding to the laminin receptor was identified and shown to have the capacity to inhibit metastasis.

### MAM (HUGO nomenclature SCGB2A2)

SCGB2A2 [7], widely known as mammaglobin or secretoglobin, family 2A, member 2, is a member of the secretoglobin subfamily [8], a group of small, secretory, rarely glycosylated, dimeric proteins mainly expressed in mucosal tissues, and that could be involved in signalling, the immune response, chemotaxis [9] and possibly, as a carrier for steroid hormones in humans.

SCGB2A2 expression has rarely been found in healthy individuals. Thus, it has become the most widely studied marker in DTC detection after CK19, at least in breast cancer patients. At the same sensitivity as CK19 [10], patients are identified with 100% specificity. Nevertheless, mammaglobin expression is highly variable in female cancers and is detected in the blood of only 10 to 30% breast cancer patients. Unfortunately, the most aggressive, steroid receptor-negative, high-grade breast tumors and their corresponding CTC are likely to escape detection using SCGB2A2 as marker.

Zafrakas et al [11] found SCGB2A2 abundantly expressed in tumors of the female genital tract, i.e. endometrial, ovarian and cervical cancer. This observation might extend the diagnostic potential of SCGB2A2 to the detection of CTC from gynecologic malignancies.

### TFF1

Members of the trefoil family are characterized by having at least one copy of the trefoil motif, a 40-amino acid domain that contains three conserved disulfides. They are stable secretory proteins expressed in gastrointestinal mucosa. Their functions are not defined, but they may protect the mucosa from insults, stabilize the mucus layer, and affect healing of the epithelium. This gene, which is expressed in the gastric mucosa, has also been studied because of its expression in human tumors. This gene and two other related trefoil family member genes are found in a cluster on chromosome 21. TFF1 expression is correlated with steroid receptor status and elevated transcript levels have been observed in various neoplastic tissues, including breast cancer [12].

### BAIAP2L1

This gene is located on 7q22.1 and encodes a member of the IMD (IRSp53/MIM homology domain) family. Members of this family can be subdivided in two groups, the IRSp53-like and MIM-like, based on the presence or absence of the SH3 (Src homology 3) domain. The protein encoded by this gene contains a conserved IMD, also known as F-actin bundling domain, at the N-terminus, and a canonical SH3 domain near the C-terminus, so it belongs to the IRSp53-like group. This protein is the substrate for insulin receptor tyrosine kinase and binds to the small GTPase Rac. It is involved in signal transduction pathways that link deformation of the plasma membrane and remodelling of the actin cytoskeleton. It also promotes actin assembly and membrane protrusions when overexpressed in mammalian cells, and is essential to the formation of a potent actin assembly complex during EHEC (Enterohemorrhagic Escherichia coli) pedestal formation.

### ESRP2

ESPR2 is an epithelial cell-type-specific splicing regulator. Epithelial- and mesenchymal- specific isoforms that are regulated by the ESRPs are likely to participate in epithelial-mesenchymal crosstalk during early vertebrate development and to have important roles in epithelial to mesenchymal transitions during development as well as in disease processes such as cancer metastasis and tissue fibrosis [13].

### EPCAM (Epithelial cell adhesion molecule)

This gene encodes a carcinoma-associated antigen and is a member of a family that includes at least two type I membrane proteins. This antigen is expressed on most normal epithelial cells and gastrointestinal carcinomas and functions as a homotypic calcium-independent cell adhesion molecule. Because of its ubiquitous expression on the surface of epithelial cells, *EPCAM* can be considered as a pan-carcinoma tumor marker. The antigen is used as a target for immunotherapy treatment of human carcinomas.

*EPCAM* has been frequently used as target for positive immunomagnetic separation to enrich tumor cells for RT-PCR analysis. Monoclonal antibodies against this antigen have been extensively developed for diagnostic (CellSearch), but also therapeutic, approaches. Although highly sensitive for epithelial malignancies, including breast cancer, however, its use for CTC detection is hampered by the fact that it is expressed in low amounts in peripheral blood cells. Furthermore, it has been shown that the normal-like breast cancer cells characterized by aggressive behaviour and worse treatment options are not recognized by the CellSearch test (Veridex, LLC), which is the only diagnostic test for circulating tumor cells currently approved by the US Food and Drug Administration and which utilizes an anti-EpCAM antibody.

According to a specific aspect there is preferably provided a set of reagents to determine the biomarker according to the invention, in particular a set including reagents that specifically react with the biomarker or the biomarker expression.

Preferably, means for determining a biomarker or the expression pattern or expression signature according to the invention are employed to provide a multi-marker panel, comprising at least two biomarkers according to the invention, comprising PPIC and optionally CDH3 and further biomarkers, which may be particularly used for determination of ovarian tumor disease. According to a specific aspect the multimarker panel is particularly comprising or consisting of ovarian cancer markers. Thus, an ovarian cancer multi-marker panel is used, in particular with a limited number of markers, e.g. up to 50, preferably up to 40 or up to 30, or up to 20 or up to 15 markers.

This panel according to the invention preferably further comprises one or more markers selected from the group consisting of GPX8, TUSC3, AGR2, COL3A1, LAMB1, MAM (SCGB2A2), TFF1, BAIAP2L1, ESRP2 and EpCAM.

In a specific embodiment, the invention contemplates marker panels containing or consisting essentially of at least two, three, four, five, six, or more, wherein at least one of the biomarkers is PPIC. The inventive panel preferably includes only those biomarkers that are associated with ovarian cancer, preferably only those that would differentiate between patients having detectable CTCs associated with malignancy or metastasis and healthy subjects, which eventually have non-tumor derived cells in a body fluid sample. The multimarker panel preferably comprises the biomarker polypeptide or gene sets.

The term "subject" as used herein shall refer to any mammal, in particular a human but also selected from animals, such as those used for tumor models and other animal studies. Preferably, the subject shall be human beings, in particular female, who are patients at risk of an ovarian tumor, in particular malignant lesions The term "patient" herein always includes healthy subjects.

A subject "at risk of" tumor or cancer disease is herein understood as a subject that has an already diagnosed or undiagnosed tumor or cancer, including those already suffering from such a disease at various stages, including the early stage and advanced disease state, particularly associated with malignant tumors, or else subjects that develop a progressive disease. In accordance therewith, the determination of the risk of ovarian cancer is herein specifically referring to diagnosis, prognosis and/or prediction of therapy response, including monitoring the disease.

The risk determination to diagnose ovarian cancer is particularly important in a subject, where the ovarian malignancy has not yet been diagnosed. This risk determination therefore includes early stage diagnosis. Preferably, those patients are tested for the biomarker according to the invention, before a solid tumor is detected, or before malignancy has proven by biopsy, where no cancerous disease is diagnosed.

Healthy subjects are usually not tested for any tumor disease biomarkers in the absence of any detectable tumor. However, there are high risk subjects, who have an increased risk of developing a gynecologic tumor and specifically ovarian cancer disease because of a genetic predisposition or other risk factors, including age, life style, family predisposition or history. Antecedent diseases, such as cancer, or benign tumors or certain medical treatment would also increase the risk of developing solid tumors and associated disease conditions. Several risk factors for solid tumors that classify a high cancer risk, e.g. breast cancer, have been identified so far, among them BRCA1-, BRCA2-, p53- gene mutations, hormonal therapies, etc.

The early detection of solid tumor disease is essential in the patient population that is already classified as high-risk patients. It is thus preferred to test a patient population according to the invention, which is already classified as risk patients.

In particular, the inventive method allows the early stage determination of the solid tumor disease or respective risk stages, e.g. to distinguish between low, medium and high risk patients.

In advanced cancer disease but also in minimal residual disease, the risk of relapse can be high, which is usually associated with poor prognosis. Thus, the risk determination according to the invention particularly refers to the prognosis of a subject to develop cancer relapse and/or the prognosis of a cancer patient, and in particular to the determination of the metastatic potential.

The method according to the invention is specifically provided for determining susceptibility to ovarian cancer or the risk of ovarian cancer disease, in a subject comprising:
(a) providing a blood sample from a subject,
(b) detecting or identifying in the sample PPIC and eventual further biomarkers of the panel of the invention, and
(c) comparing the detected amount with a standard amount or an amount detected for a reference.

The term "detect" or "detecting" includes assaying, imaging or otherwise establishing the presence or absence of the target biomarker or a combination of biomarkers of a specific panel. The level of biomarkers or amount of biomarkers is herein understood to refer to the respective polypeptides or nucleotide sequence, including variants such as splice variants, subunits thereof, or reagent bound targets.

The target biomarker is preferably determined by testing for the respective polypeptides and/or polynucleotides indicative of marker expression. The expressed marker is detectable e.g. as polynucleotide, like mRNA, or expressed polypeptide or protein. The comparison with the reference value should be of the same sample type. Thus, the reagents preferably comprise ligands specifically binding to the biomarker polypeptide or gene or genetic marker, e.g. comprising a plurality of respective polypeptides, genes or polynucleotides. Ligands are herein understood as marker specific moieties.

Marker specific moieties are substances which can bind to or detect at least one of the markers for a detection method descried above and are in particular marker nucleotide sequence detecting tools or marker protein specific antibodies, including antibody fragments, such as Fab, F(ab), F(ab)', Fv, scFv, or single chain antibodies. The marker specific moieties can also be selected from marker nucleotide sequence specific oligonucleotides, which specifically bind to a portion of the marker sequences, e.g. mRNA or cDNA, or are complementary to such a portion in the sense or complementary anti-sense, like cDNA complementary strand, orientation.

The preferred ligands may be attached to solid surfaces, including beads, to catch and separate the marker or CTC in the sample, and/or to labels. Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, etc.) as well as molecules that may be indirectly detected by production of a detectable reaction product (e.g., enzymes such as horseradish peroxidase, alkaline phosphatase, etc.) or by a specific binding molecule which itself may be detectable (e.g., biotin, digoxigenin, maltose, oligohistidine, 2,4-dintrobenzene, phenylarsenate, ssDNA, dsDNA, etc.).

In particular aspects of the invention, the methods described herein utilize PPIC and optionally one or more markers of a multimarker panel placed on a microarray so that the expression status of each of the markers is assessed simultaneously. In an embodiment, the invention provides a microarray as a prognostic tool comprising a defined set of marker genes, whose expression is significantly altered in gynecologic and specifically ovarian cancer and which may be determined by hybridization or by amplification of polynucleotides.

In preferred embodiments, the mRNA concentration of the marker(s) is determined. To this extent, mRNA of the sample can be isolated, if necessary, after adequate sample preparation steps, e.g. tumor cell enrichment and/or lysis, and hybridized with marker specific probes, in particular on a microarray platform with or without amplification, or primers for PCR-based detection methods, e.g. PCR extension labelling with probes specific for a portion of the marker mRNA.

The invention preferably contemplates a gene expression profile comprising a multimarker panel that is associated with ovarian cancer, including the PPIC marker. This profile provides a highly sensitive and specific test with both high positive and negative predictive values permitting diagnosis and prediction of the patient's risk or the risk of developing metastatic disease.

For example, the invention provides a method for determining the risk of ovarian cancer disease in a subject comprising
(a) contacting a blood sample obtained from said subject with one or more oligonucleotides that hybridize with one or more markers, which are PPIC and optionally one or more of the markers of the multimarker panel described above, and
(b) detecting in the sample a level of polynucleotides that hybridize to the one or more markers relative to a reference level or predetermined cut-off value, and therefrom determining the risk of ovarian cancer or the risk of developing ovarian cancer in the subject.

Within certain preferred embodiments, the amount of mRNA is detected via polymerase chain reaction using, for example, oligonucleotide primers that hybridize to a marker gene, or complements of such polynucleotides. When using mRNA detection, the method may be carried out by combining isolated mRNA with reagents to convert to cDNA according to standard methods and analyzing the products to detect the marker presence in the sample.

In further embodiments the amount of a marker or any combination thereof is determined by the polypeptide or protein concentration of the marker(s), e.g. with marker specific ligands, such as antibodies or specific binding partners. E.g., the binding event can be detected by competitive or non-competitive methods, including the use of labeled ligand or marker specific moieties, e.g. antibodies, or labeled competitive moieties, including a labeled marker standard, which compete with marker proteins for the binding event. If the marker specific ligand is capable of forming a complex with the marker, the complex formation indicates expression of the markers in the sample.

In particular, the invention relates to a method for diagnosing and monitoring ovarian cancer disease in a patient by quantitating a marker in a blood sample from the patient comprising
(a) reacting the sample with one or more binding agents specific for PPIC and optionally one or more markers of the multimarker panel according to the invention, e.g. an antibody or antibody fragment that is directly or indirectly labelled with a detectable substance, and
(b) detecting the detectable substance.

The preferred method employs an immunoassay. In general, immunoassays involve contacting a sample potentially containing a biomarker of interest with at least one immunoligand that specifically binds to the marker. A signal is then generated indicative of the presence or amount of complexes formed by the binding of polypeptides in the sample to the immunoligand. The signal is then related to the presence or amount of the marker in the sample. Immunoassays and respective tools for determining PPIC and the other markers are well-known in the art.

Described are also kits for carrying out the methods of the invention, specifically those including a reference, training data or a standard.

The invention further contemplates the methods, compositions, and kits described herein using additional markers associated with ovarian cancer. The methods described herein may be modified by including reagents to detect the additional markers, or polynucleotides for the markers.

Preferably, the differential marker expression is determined by comparing the expression to the control of healthy subjects or patients suffering from a benign tumor.

Reference values for the biomarker are preferably obtained from a control group of subjects with normal expression of said biomarker, or a biomarker expression, that is associated with the disease condition, such as disease stages, which represents the appropriate reference value. In a particular aspect, the control comprises material derived from a pool of samples from normal subjects. The normal level of a biomarker may be determined in samples of the same type obtained from control subjects.

The reference values are typically calculated from standard deviations of the mean average marker expression in healthy subjects. If more than one marker is detected, the comparison is made to each single reference value for each marker in the reference itself. The risk of the presence of a gynecologic tumor or ovarian cancer may be indicated if the amount of the biomarker or the combination of markers is significantly increased as compared to a standard, e.g. derived from a non-tumor cell, or reference value of subjects not suffering from gynecologic tumor or ovarian cancer, preferably being subjects from a control group or healthy subjects. The risk determination typically correlates with the level of overexpression. If at least two biomarkers of the panel according to the invention are increased, the risk is considered to be even more increased. With regard to the PPIC and the other markers, overexpression indicates an increased risk of the ovarian cancer disease. However, an increase of CDH3 overexpression may indicate a prolonged survival.

The marker level can also be compared to a threshold, e.g. a cut-off concentration and the likelihood for the presence of ovarian cancer disease is determined from such comparison; wherein the PPIC concentration above the reference value is predictive of cancer disease or disease development or progression in the patient.

In a preferred quantitative determination method, the expression of biomarkers is normalized to the median expression of one or more reference genes, used as internal control.

Thus, the preferred method according to the invention comprises the step of comparing the PPIC marker level with a predetermined standard or cut-off value, which is preferably at least 25% higher than the standard, more preferred at least 40% or 50% higher, but can also be at least 100% higher.

In terms of overexpression of a biomarker according to the invention, it is contemplated that the levels of the biomarker expression in a sample of a patient diagnosed with metastatic ovarian cancer are higher than in a standard or reference representing patients diagnosed with non-metastatic disease, and the levels of the biomarker expression in a sample of a patient diagnosed with ovarian cancer are higher than in a standard or reference representing subjects free of ovarian cancer or any malignancy. Specifically, the PPIC determination according to the invention provides a valuable contribution as an indicator of ovarian cancer disease and disease progression.

Indicators or predictive values help interpret the results of tests in the clinical setting. The diagnostic value of the method according to the invention is primarily defined by its sensitivity, specificity, predictive value and efficiency. Any test method will produce true positive, false negative, false positive, and true negative, which are all factors determining the sensitivity or specificity of a test, thus determining a likelihood to support a diagnosis or determination of a disease state and development. The higher the likelihood, the better the test can support a diagnosis.

Diagnosis can be obvious in patients with combinations of classic manifestations. However, in some patients, the diagnosis cannot be made clinically, and confirmatory laboratory tests can increase the probability of disease but do not rule it out. Thus, the PPIC determination according to the invention is considered an invaluable tool indicating ovarian cancer disease and disease progression.

The higher the fold increase of PPIC, the higher is the patient's risk of ovarian cancer disease. An elevated PPIC value alone or in combination with the other markers of the panel according to the invention indicates, for example, special treatment of the patient, using appropriate medication or further diagnostic techniques, such as imaging and surgical interventions. The method of the invention can thus be used to evaluate a patient before, during, and after medical treatment.

Types of cancer treatment that are used as adjuvant therapy include chemotherapy, hormone therapy, radiation therapy, immunotherapy or targeted therapy. Following first line chemotherapy, for instance, the cancer patient can be determined for the metastatic potential to decide about a second line adjuvant treatment.

According to a specific embodiment, the numbers of CTC in blood is determined for diagnostic or prognostic purposes. The CTC may be enriched in a body fluid, in particular blood, and the expression profile of the cells is determined. When a ligand specifically binding to the biomarker is used as capturing agent, the CTCs are preferably enriched and optionally isolated before testing the marker expression, and the test results are evaluated, e.g. according to the CTC epithelial cell functions or properties.

The enrichment of CTC is particularly preferred for genome analysis or molecular analysis employing nucleic acids as probes to hybridize with the specific biomarkers. For instance, RT-PCR or RT-qPCR is preferably employed. Upon enrichment of CTC, the RNA can be analyzed. For example, disseminated, circulating tumor cells from peripheral blood are enriched using a cell separation procedure to produce a fraction prior to sample analysis. A standardized system for tumor cell enrichment is e.g. provided as OncoQuick® (Greiner Bio-One, Frickenhausen, Germany). Thereby a fraction of mononuclear cells is obtained, optionally co-enriched with tumor cells and subsequent nucleic acid or immunocytochemical evaluation is possible with high sensitivity.

The invention also contemplates a method of assessing the potential of a test compound to contribute to ovarian cancer therapy. For instance, an *ex vivo* method according to the invention may comprise the following steps:
(a) maintaining separate aliquots of a blood sample from a patient in the presence and absence of the test compound, and
(b) comparing the levels of PPIC and optionally of one or more further biomarkers, e.g. a multimarker panel in each of the aliquots.

This method may also be particularly useful as an *in vivo* method in monitoring the marker level in non-human animal models, or during clinical trials. A significant difference between the levels of a marker in an aliquot maintained in the presence of or exposed to the test compound relative to the aliquot maintained in the absence of the test compound, indicates that the test compound potentially contributes to ovarian cancer therapy.

The present invention is further illustrated by the following example without being limited thereto.

### Example 1: Prognosis of ovarian cancer patients

In the first step, a whole genome expression analysis of ovarian cancer tissues (35 patients) and of peripheral blood mononuclear cells (20 healthy female donors, 35 patients) using microarrays was performed. From 40 gene markers with low/absent gene expression PBMCs and high/present gene expression in ovarian cancer tissues 25 markers were selected for further analysis. Additionally, 15 markers were selected due to results of a previous study aiming at defining molecular markers for the detection of CTCs in breast cancer and gynecologic cancer (ovarian, endometrial and cervical cancer).

As a reference, the gene expression of the 40 differentially expressed genes was validated in PBMCs obtained from 20 patients with benign ovarian diseases with RT-qPCR. As a result, 11 gene markers remained in the study due to absent/low gene expression in benign PBMCs.

Then the gene expression of these 11 markers was analysed in the blood of 248 ovarian cancer patients and 39 healthy females. EpCAM (Epithelial cell adhesion molecule) was analysed as additional gene marker to compare results obtained from RT-qPCR and from immunocytochemistry. 230 blood samples were taken before initial surgery of the primary tumor, and 115 blood samples six months after completion of the first line adjuvant chemotherapy. From 97 patients blood samples taken at broth timepoints were available.

Gene expression was normalized to the median expression of three reference genes (B2M, ACTB, and TBP). Due to background gene expression in the healthy PBMC samples, a threshold TX was introduced to separate healthy and diseased individuals and to identify CTC-positive patients as proposed by Mikhitarian et al. (BMC Cancer 2008, 8:55). A patient was defined as CTC-positive if an at least one out of 11 gene marker over-expressed.

As a result, 56/230 patients were positive before initial surgery, but only 21/115 six months after chemotherapy (Table 1). Detailed results for the respective gene markers are shown in Table 2. The presence of CTC as indicated by RT-qPCR was correlated to clinical parameters of the patients (age, response to adjuvant chemotherapy, residual tumor after surgery, FIGO, peritonealcarcinomatosis, ascites, and recurrence during follow-up). Significantly more patients were CTC positive before initial surgery, who had ascites or had residual tumor masses after surgery (Table 3). Six months after completion of their adjuvant chemotherapy, significantly more FIGO IV than FIGO II or III patients and significantly more patients who did not respond to the treatment were CTC positive (Table 4). The presence of CTC before initial surgery had no influence on patients' survival. Patients still presenting CTC six months after chemotherapy had a significantly shorter overall and progression free survival (patients were followed for a median of 31 months).

Results obtained with RT-qPCR and immunocytochemistry perfectly agreed in 35/57 (61%) blood samples (53% both negative, 9% both positive). 15/57 (26%) samples were identified by ICC as CTC-positive, but not with RT-qPCR. 7/57 (12%) samples were only identified by RT-qPCR. Interestingly, patients with CTC identified by RT-qPCR only had a significantly worse prognosis than patients with both RT-qPCR- and ICC-negative results.

Cyclophilin C (PPIC) was predominantly over-expressed in the blood of CTC+ ovarian cancer patients. These patients had a significantly shorter overall and progression free survival compared to those CTC+ patients without over-expression. In contrast, p-Cadherin (CDH3) gene expression had a reverse impact on patient outcome. PPIC and CDH3 gene expression might characterize CTC with different metastatic potential.

The multiple survival analysis using a proportional hazards Cox regression model including age, FIGO stage, residual tumor, grade, and marker gene over-expression before and after treatment revealed that stage FIGO IV, peritonealcarcinomatosis, and the presence of circulating tumor cells as identified by RT-qPCR after chemotherapy are independent predictors of reduced progression free survival. Patient age, peritonealcarcinomatosis, and circulating tumor cells still present after chemotherapy are independant predictors of reduced overall survival (Table 5).

In summary, starting from a whole genome expression analysis of ovarian cancer tissue samples and peripheral blood mononuclear cells we identified 11 novel gene marker for the detection and characterization of circulating tumor cells in the blood of ovarian cancer patients. Using this gene panel for multimarker RT-qPCR analysis, CTC were detected in blood samples taken before initial treatment in 24% of the patients. The presence of CTC six months after completion of chemotherapy reflected worse patient outcome. The molecular detection of CTC-provided additional information where standard immunocytochemistry failed. The over-expression of two genes (*PPIC* and *CDH3*) has opposing impact on patient outcome. Therefore, *PPIC* and *CDH3* gene expression might characterize CTC with different metastatic potential.

**Table 1: CTC before initial surgery and six months after chemotherapy**

| | | Number of Patients (%) | |
|---|---|---|---|
| number of overexpressed gene markers | | Before initial surgery | 6 months after chemo |
| | ≥ 1 | 56 (24.3) | 21 (18.3) |
| | 1 | 42 (18.3) | 18 (15.7) |
| | 2 | 8 (3.5) | 3 (1.2) |
| | 3 | 2 (0.8) | |
| | 4 | 1 (0.4) | |
| | 5 | 1 (0.4) | |
| | 6 | 2 (0.8) | |

**Table 2: Overexpression of 11 gene markers and of EpCAM before initial surgery and six months after chemotherapy**

| Gene symbol | Gene name | Before initial surgery | | | 6 months after chemo | | |
|---|---|---|---|---|---|---|---|
| | | *N* | % | % pos | *N* | % | % pos |
| *PPIC* | Cyclophilin C | 39 | 17.0 | 69.6 | 15 | 13.0 | 71.4 |
| *GPX8* | Probable glutathione peroxidase 8 | 14 | 6.1 | 25.0 | 1 | 0.9 | 4.8 |
| *CDH3* | Cadherin-3 | 9 | 3.9 | 16.1 | 2 | 1.3 | 9.5 |
| *TUSC3* | Tumor suppressor candidate 3 | 7 | 3.0 | 12.5 | 1 | 0.9 | 4.8 |
| *AGR2* | Anterior gradient protein 2 homolog | 1 | 0.4 | 0.2 | 1 | 0.9 | 4.8 |
| *COL3A1* | Collagen alpha-1(III) chain | 3 | 1.3 | 5.4 | 1 | 0.9 | 4.8 |
| *LAMB1 TFF1* | Laminin subunit beta-1 | 3 | 1.3 | 5.4 | 1 | 0.9 | 4.8 |
| *MAM* | Mammaglobin A | 2 | 0.9 | 3.6 | 0 | | |
| | Trefoil factor 1 | 1 | 0.4 | 0.2 | 0 | | |
| *BAIAP2L1* | Brain-specific angiogenesis inhibitor 1-associated protein 2-like protein 1 | 0 | | | 0 | | |
| *ESRP2* | Epithelial splicing regulatory protein 2 | 2 | 0.9 | 3.6 | 1 | 0.9 | 4.8 |
| *EPCAM* | Epithelial cell adhesion molecule | 4 | 1.7 | 7.1 | 1 | 0.9 | 4.8 |

**Table 3: Correlation of CTC presence before initial surgery with clinical parameters**

| | Number of patients (%) | | p-value |
|---|---|---|---|
| | CTC positive | CTC negative | |
| Total | 56 (24) | 174 (76) | |
| Mean age (yrs) | 59.2 (±12.7) | 58.4 (±11.5) | n.s. |
| FIGO stage | | | |
| II/III (N=191) | 45 (24) | 146 (76) | n.s |
| IV (N=39) | 11 (28) | 28 (72) | |
| Therapy response | | | |
| yes (N=172) | 41 (24) | 131 (76) | n.s |
| no (N=57) | 15 (26) | 42 (74) | |
| Subsequent recurrence | | | |
| yes (N=148) | 39 (26) | 109 (74) | n.s |
| no (N=82) | 17 (21) | 65 (79) | |
| Residual disease | | | |
| yes (N=73) | 24 (33) | 49 (67) | 0.032 |
| no (N=156) | 31 (20) | 125 (80) | |
| Peritonealcarcinomatosis | | | |
| yes (N=155) | 41 (27) | 114 (73) | n.s. |
| no (N=74) | 14 (19) | 60 (81) | |
| Ascites | | | |
| yes (N=173) | 49 (28) | 124 (72) | 0.014 |
| no (N=57) | 7 (12) | 50 (88) | |

**Table 4: Correlation of CTC presence 6 months after chemotherapy with clinical parameters**

| | Number of patients (%) | | p-value |
|---|---|---|---|
| | CTC positive | CTC negative | |
| Total | 21 (18) | 94 (82) | |
| Mean age (yrs) | 63.8 (±12.2) | 56.1 (±11.3) | 0.007 |
| FIGO stage | | | |
| II/III (N=99) | 14 (14) | 85 (86) | 0.01 |
| IV (N=16) | 7 (44) | 9 (66) | |
| Therapy response | | | |
| yes (N=95) | 13 (14) | 82 (86) | 0.01 |
| no (N=20) | 8 (40) | 12 (60) | |
| Subsequent recurrence | | | |
| yes (N=76) | 17 (22) | 59 (78) | n.s. |
| no (N=39) | 4 (10) | 35 (90) | |
| Residual disease | | | |
| yes (N=84) | 15 (18) | 69 (82) | n.s |
| no (N=30) | 6 (20) | 24 (80) | |
| Peritonealcarcinomatosis | | | |
| yes (N=78) | 18 (23) | 60 (77) | n.s |
| no (N=36) | 3 (8) | 33 (92) | |
| Ascites | | | |
| yes (N=92) | 16 (17) | 76 (83) | n.s |
| no (N=23) | 5 (22) | 18 (78) | |

**Table 5: Cox's proportional hazard regression models for progression free and overall survival**

| | Univariate Cox-Regression | | | Multiple Cox-Regression | | |
|---|---|---|---|---|---|---|
| | HR | CI 95% | p | HR | CI 95% | p |
| Age | 1.012 | 0.998-1.026 | 0.102 | | | |
| FIGO IV vs. II/III | 2.269 | 1.550-3.323 | <0.001 | 2.725 | 1.436-5.168 | 0.002 |
| Residual tumor yes vs. no | 2.117 | 1.527-2.934 | <0.001 | | | |
| Grade 3 vs. 1/2 | 1.525 | 1.058-2.198 | 0.024 | | | |
| Peritonealcarcinomatosis yes vs. no | 3:112 | 2.105-4.600 | <0.001 | 3.550 | 1.946-6.478 | <0.001 |
| Ascites yes vs.no | 1.443 | 0.975-2.135 | 0.067 | | | |
| CTC before surgery | 1.325 | 0.918-1.912 | 0.133 | | | |
| CTC after chemo | 2.268 | 1.312-3.922 | 0.003 | 2.063 | 1.175-3.619 | 0.012 |

### Methods

### Selection of candidate genes

Microarray data was obtained from ovarian cancer tissues (35 patients) and from peripheral blood mononuclear cells (20 healthy female donors, 35 patients). 25 markers with low/absent gene expression in healthy PBMCs and high expression in ovarian cancer tissues were selected as candidate gene markers for the detection of circulating tumor cells in the blood of ovarian cancer patients. 15 additional markers were selected from results generated in a previous national project. The expression of these 40 differentially expressed genes was validated in PBMCs obtained from 20 patients with benign ovarian diseases with RT-qPCR. 11 gene markers that had no expression in the benign PBMC remained for further analysis. EpCAM (Epithelial cell adhesion molecule) was analysed as additional gene marker to compare results obtained from RT-qPCR and from immunocytochemistry.

### Blood sample preparations

25 ml peripheral blood was drawn in Vacuette EDTA tubes and processed within 30 minutes. The monocyte blood fraction containing epithelial cells was enriched using a density gradient centrifugation as described [1]. After two washings with each 50 ml PBS, the cell pellet was lysed in 350 µl RLT buffer (Qiagen, Hilden, Germany). Automated purification of RNA using the RNeasy Mini Kit was performed by the Qiacube system (Qiagen, Hilden, Germany). Quality of the RNA samples was assessed on the 2100 Bioanalyzer using the RNA 6000 Nano LabChip Kit (Agilent Technologies, Waldbronn, Germany). Template cDNA was generated with M-MLV Reverse Transcriptase, RNase H Minus (Promega, Madison WI, USA) and random nonamers (Sigma-Aldrich, Steinheim, Germany) as primers.

### RT-qPCR

RT-qPCR was performed on the 7900HT Fast Real-Time PCR System in duplicate reactions using TaqMan® Pre-Developed Assay Reagents (see Table 6) and TaqMan® Universal PCR Master Mix, No AmpErase® UNG (all purchased from Applied Biosystems, Foster City CA, USA) as recommended by the manufacturer. Raw data were analyzed with the AB 7900 Sequence Detection Software version 2.2.2 using automatic baseline correction and cycle threshold (Ct) setting. Gene expression was normalized to the geometric mean of the endogenous control gene expression levels (GAPDH, ACTB, and TBP). Low-level gene expression in the healthy control samples required the introduction of a cut-off threshold value to separate the cancer patient group from the healthy control group:
As proposed by Mikhitarian et al. [2], a threshold value TX for each gene X was set to three standard deviations from the mean dCtX value in the control group. A tumor patient was considered positive for the molecular analysis of gene X if dCtX was below the defined threshold value TX.

**Table 6: TaqMan Assay IDs**

| | |
|---|---|
| AGR2 | Hs00180702_m1 |
| BAIAP2L1 | Hs00218959_m1 |
| CDH3 | Hs00354998_m1 |
| COL3A1 | Hs00164103_m1 |
| ESRP2 | Hs00227840_m1 |
| GPX8 | Hs00380670_m1 |
| LAMB1 | Hs00158620_m1 |
| PPIC | Hs00181460_m1 |
| SCGB2A2 | Hs00267190_m1 |
| TACSTD1 | Hs00158980_m1 |
| TFF1 | Hs00170216_m1 |
| TUSC3 | Hs00954406_m1 |
| EpCAM | Hs00158980_m1 |

### Example 2: Molecular characterization of circulating tumor cells in patients with ovarian cancer improves their prognostic significance

Following Example 1, the study was continued confirming the relevance of PPIC and other biomarkers in the peripheral blood of ovarian cancer patients. Details of the extended study are provided below.

### Purpose

The study aims at identifying novel markers for circulating tumor cells (CTC), at applying these markers in patients with epithelial ovarian cancer (EOC), and at evaluating the impact of CTC on patient outcome.

### Experimental design

Microarray analysis comparing matched EOC tissues and peripheral blood leucocytes (N=35) was performed. After a confirmatory RT-qPCR step, 11 genes were identified as novel markers for the detection of CTC. Blood samples were taken from EOC patients (N=216) before primary treatment and six months after adjuvant chemotherapy, and mRNA levels of the 11 genes and of EpCAM were analyzed after enrichment by density gradient centrifugation. CTC positivity was defined by over-expression of at least one gene as compared to the healthy control group (N=39).

### Results

CTC were detected in 24.5% of the baseline and 20.4% of the follow-up samples. PPIC was the most frequently over-expressed gene marker (17% baseline, 14% followup). Presence of CTC at baseline correlated with the presence of ascites (p=0.021) and suboptimal debulking (p=0.013), whereas CTC during follow-up occurred more often in patients with older age (p=0.016), higher FIGO stage (p=0.035), and chemoresistance (p=0.035). PPIC positive CTC were independent prognostic indicators for shorter DFS (HR=1.643, p=0.031) and OS (HR=2.229, p=0.010) at baseline, and for shorter DFS during follow-up (HR=3.415, p<0.001).

### Conclusion

Molecular characterization of CTC is superior to a mere CTC enumeration or even be the rationale for CTC diagnostics at all. Ultimately CTC diagnostics may lead to more personalized treatment of EOC, especially in the recurrent situation.

### Material and Methods

Patients, who were undergoing evaluation for suspected primary EOC from January 2006 to December 2008 were recruited in five European clinical centers (Department of Gynecology, European Competence Center for Ovarian Cancer; Campus Virchow Klinikum, Charité-Universitätsmedizin Berlin (D), Division of Gynaecological Oncology, Department of Obstetrics and Gynaecology, Universitaire Ziekenhuizen Leuven, Katholieke Universiteit Leuven (B), Department of Gynecology and Gynecologic Oncology, University Medical Center Hamburg-Eppendorf (D), Department of Obstetrics and Gynecology, Innsbruck Medical University (A), and Department of Obstetrics and Gynecology, Medical University of Vienna (A)). All histological types of EOC were included. Patients with benign ovarian diseases, borderline ovarian cancer, secondary malignant tumors, or with FIGO stage I ovarian cancer were excluded. The patients received standard treatment including debulking surgery and platinum-based chemotherapy. Response to chemotherapy was evaluated according to the WHO criteria, i.e. progression of disease after first-line chemotherapy was defined by an increase in the nadir serum CA-125 level of at least two folds according to the GCIG criteria or by radiological (clinical) confirmation. The response to first-line treatment was evaluated by experienced gynecological oncologists of the participating university centers. Additionally, 39 healthy female volunteers without any history of cancer were recruited as negative control group. All patients and healthy volunteers gave written informed consent. The study was approved by the Ethics Committees of the participating OVCAD partners (EK207/2003, ML2524, HEK190504, EK366, EK260).

### Blood sample collection and preparation

Twenty-five ml of peripheral blood was collected before primary surgery or neoadjuvant chemotherapy (i.e. baseline samples), and six months ± 21 days after completion of the standard platinum-based chemotherapy (i.e. follow-up samples). The blood samples were drawn in Vacuette EDTA tubes and processed within two hours. The monocyte blood fraction possibly containing epithelial cells, called "CTC fraction" was enriched using a two-layer density gradient centrifugation as described previously. After two washing steps with each 50 ml PBS, the cell pellet was lysed in 350 µl RLT buffer. (Qiagen, Hilden, D). Fully automated purification of RNA using the RNeasy Mini Kit was performed by the Qiacube system (Qiagen, Hilden, D). The quality of the RNA samples was assessed on the 2100 Bioanalyzer using the RNA 6000 Nano LabChip Kit (Agilent Technologies, Waldbronn, D). Samples with a RNA integrity number smaller than 5 were excluded from further analysis. For reverse transcription, 200 pmol random nonamers (Sigma-Aldrich, Steinheim, D) were added to the RNA samples and incubated at 70°C for 5 min. First strand cDNA synthesis was performed in a mix containing 160 U M-MLV Reverse Transcriptase, RNase H Minus, Point Mutant, 10 U RNasin® Plus RNase Inhibitor (all purchased from Promega, Madison WI, USA), 500 µM of an equimolar mix of dATP, dCTP, dTTP and dGTP (Amersham Biosciences, Freiburg, D), 50 mM Tris-HCl (pH 8.3), 75 mM KCl, 3 mM MgCl2 and 10 mM DTT. The reaction was incubated at 25°C for the initial 15 min., then 45°C for 1 hour, and 55°C for the final 10 minutes.

### Microarray analysis and marker preselection

Whole genome expression analysis was performed with Human Genome Survey Microarrays V2.0 (Applied Biosystems, Foster City Ca, USA) containing 32,878 probes representing 29,098 genes. 20 µg total RNA from leukocyte samples (N=20 healthy females) and from paired fresh frozen tumor tissues and leukocyte samples (N=35 EOC patients) were labeled with the Chemiluminescent RT Labeling Kit (Applied Biosystems) and hybridized to the microarrays for 16 hours at 55°C. After washing and visualization of bound digoxigenin labeled cRNAs with the Chemiluminescence Detection Kit according to the manufacturer's instructions (Applied Biosystems), images were read with the 1700 Chemiluminescent Microarray Analyzer (Applied Biosystems). Raw expression data, signal-to-noise ratios (S/N) and quality-flags delivered from the Applied Biosystems Expression System software were further processed using Bioconductor's ABarray package. Raw expression values were log2 transformed, and measurements with quality indicator flag values greater than 5000 were set missing. Finally, 25 genes with present calls (S/N>3) in more than 90% of the tissue samples and absent calls (S/N<2) in more than 75% of the blood samples were selected as putative CTC markers. Additionally, we decided to include further 15 gene markers selected previously. The gene expression levels were evaluated with RT-qPCR in "CTC fractions" of 20 patients with benign ovarian disease, who were supposed to be free of CTC. Genes with absent or marginal expression levels in the benign blood samples were selected as candidate markers for the detection of CTC in cancer patients.

### RT-qPCR

RT-qPCR was performed on the 7900HT Fast Real-Time PCR System in duplicate reactions using TaqMan® Pre-Developed Assay Reagents and TaqMan® Universal PCR Master Mix, No AmpErase® UNG (all purchased from Applied Biosystems, Foster City, CA) with default thermal cycling parameters (50°C for 2 min; 95°C for 10 min., 50 cycles at 95°C for 15 s, 60°C for 1 min.). Raw data were analyzed with the AB7900 Sequence Detection Software version 2.2.2 using automatic baseline correction and manual cycle threshold setting. Gene expression was normalized to the median expression of three reference genes (B2M, ACTB, and TBP). A threshold value TX for each gene X was set to three standard deviations from the mean dCtX value in the healthy control group. A patient was defined as CTC-positive, if at least one gene marker was over-expressed beyond the defined threshold.

### Statistical analysis

The Pearson's chi-square and Fisher's exact test were used to assess the relationship between the frequency of CTC presence and clinicopathological characteristics of the patients. Kaplan-Meier survival plots were generated on the basis of CTC presence in baseline and follow-up blood samples. Survival outcomes were compared using log-rank testing. Cox proportional hazards regression was used to determine univariate and multiple hazards ratios for disease free survival (DFS) and overall survival (OS) (21). Covariates included were patient age as continuous variable, and FIGO stage, residual disease after surgery, ascites, peritoneal carcinomatosis, and the PPIC status of the patients' CTC fraction as dichotomous variables. The model was built using a forward stepwise method by entering all variables at a p value of less than 0.05 and removing them at a p value of greater than 0.10. Statistical analysis was performed by SPSS version 19.0 (SPSS Inc., Chicago, IL). The level of significance was set at p<0.05.

### Results

### Patient characteristics

Blood samples taken from 216 EOC patients were included into the analysis. Both the baseline and the follow-up blood samples were available from 77 patients. In 123 cases only the baseline samples and in 16 cases only the follow-up samples were available. Patients were followed for a median of 29 months (range: 1-49 months). During this observation time, 57 (26.3%) patients died from their disease. Progression during primary therapy or recurrence within six months after the end of chemotherapy was detected in 46 (21.3 %) of the patients (platinum resistant recurrence). Ninety-eight (45.4%) EOC patients developed recurrent disease after six months after the end of chemotherapy (platinum sensitive recurrence).

### Presence of CTC and correlation to clinicopathological characteristics

Eleven from 40 preselected gene markers remained in the study due to absent or marginal gene expression levels as detected by RT-qPCR in "CTC fractions" of blood samples from healthy individuals (Table 7). Gene expression of these 11 candidate CTC markers and of the EpCAM gene was quantified in CTC fractions of the 216 EOC patients and 39 healthy females. Just a single (2.5%) of the healthy female blood samples was CTC positive. Of the EOC patients' samples, 49 of 200 (24.5%) baseline and 19 of 93 (20.4%) follow-up samples were CTC positive (i.e. at least one gene marker over-expressed beyond the defined threshold). The presence of ascites (p=0.021) and residual disease after primary surgery (p=0.013) correlated with baseline CTC positivity. Follow-up CTC positivity was significantly correlated with age (p=0.016), higher FIGO stage (p=0.035), and resistance to platinum-based chemotherapy (p=0.035) (Table 8).

While EpCAM overexpression was detection in only a few blood samples, cyclophilin C (PPIC) was the most prominent CTC marker: 34 (17%) baseline and 13 (14%) follow-up samples showed overexpression of the PPIC gene above the set threshold (Table 7). The presence of PPIC positive CTC at follow-up was significantly more often detected in the platinum resistant than in the platinum sensitive patient group (35.7% vs. 10.1%, p=0.024).

### Impact of CTC on patient's prognosis

First we analyzed the impact of baseline CTC positivity on patients' prognosis. EOC patients with PPIC positive CTC at baseline had a significantly shorter DFS (median 14 months, log rank p=0.009) than all other EOC patients (median 21 months). PPIC negative but CTC positive and CTC negative patients (median DFS 20 and 21 months) did not show a significant difference in prognosis and were pooled for further analysis and referred to as PPIC negative patients. OS was reduced from 34 months in PPIC negative to 18 months in PPIC positive patients (75% percentile, log rank p=0.002). Kaplan-Meier survival curves for DFS and OS according to patient's PPIC status at baseline are shown in Figure 1.

Second, we analyzed the impact of follow-up CTC positivity on patients' prognosis. Again, survival correlated with over-expression of the PPIC gene. DFS was reduced from 21 months in PPIC negative to 11 months in PPIC positive patients (log rank p= 0.001), and OS from 36 to 25 months (75.0% percentile, log rank p= 0.031). Kaplan-Meier survival curves for DFS and OS according to the patient's PPIC status at follow-up are shown in Figure 1.

Third, we also asked whether the disease free survival was different in (a) patients with PPIC positive CTC at both baseline and follow-up, (b) in patients, who were CTC negative at both time points, and in patients with PPIC positive CTC only in the baseline (c) or only in the follow-up sample (d). As a result, CTC positive patients who were CTC positive six months after adjuvant treatment relapsed earlier than those patients with CTC negative follow-up blood samples (median DFS 9 vs. 21 months, p=1.164x10⁻⁴, Figure 2). The median DFS of patients without PPIC positive CTC during follow was the same independent of their CTC status at diagnosis.

Finally, we assessed the correlation between the PPIC status in addition to classical clinicopathological parameters and the patient's prognosis. In the multiple Cox regression analysis (Table 9 and 10) the presence of PPIC positive CTC detected before treatment was an independent predictor of shorter DFS (HR=1.643, p=0.031) and OS (HR=2.229, p=0.010), whereas the presence of PPIC positive CTC detected six months after chemotherapy remained independently related to DFS only (HR=3.415, p<0.001).

### Discussion

In this study, we identified a panel of 11 novel gene markers including the PPIC gene for the detection of circulating tumor cells. We showed, that the RT-qPCR based analysis of these novel markers indicate the presence of circulating tumor cells in a substantial fraction of EOC patients both, before primary therapy and during follow-up. Furthermore, we evaluated the impact of CTC on patient outcome, and we could demonstrate that a subfraction of CTC overexpression of the PPIC gene correlates with poor patient outcome independently of classical clinicopathological parameters. To our knowledge, this is the largest study, which analyzed the clinical impact of CTC in EOC detected by novel molecular gene markers.

The presence of 24% CTC in ovarian cancer, as detected in our study are in accordance with former studies: In baseline blood samples the CTC detection rate using immunocytochemical assays was 21% (8) and 19% (22). Applying the AdnaTest Breast Cancer, Aktas et al. (14) found CTC in 19% of the patients before surgery, and in 27% of the patients 4-6 months after chemotherapy, and they observed EpCAM gene expression in 31 % and 68% of the CTC positive samples at the respective time points. Poveda et al. identified ≥ 2 CTC per 7.5 ml blood in 14% of patients with relapsed ovarian cancer before second-line therapy using the EpCAM based CellSearch system (15). In patients with newly diagnosed ovarian cancer, Marth et al. detected CTC in 12% of the patients using an immunomagnetic isolation of EpCAM positive tumor cells from blood samples taken 7 to 20 days after primary surgery (16). Lately, it has been shown that EpCAM, a classical marker for cells of epithelial phenotype, might not be the best choice for the isolation or detection of CTC, at least in breast cancer. In the present study, putative CTC of epithelial origin were enriched by applying a density gradient centrifugation, which allowed the analysis of tumor cells not displaying the classical epithelial phenotype. Inconsistent with other studies based on the EpCAM protein expression of putative CTC, we found a very low EpCAM gene positivity in only 8% of baseline and in 4% in follow-up CTC positive samples; by contrast, PPIC was over-expressed in 68% of baseline and 69% of follow-up CTC positive samples (Table 7). Thus our data are in discordance with other studies based on the EpCAM expression of putative CTC This difference might be explained by either lack of EpCAM gene expression in CTC, or by a very low expression in CTC not allowing a discrimination between healthy blood samples and CTC containing blood samples. PPIC belongs to the cyclophilin family, which includes seven major isoforms in humans. The isoforms are structurally highly conserved throughout revolution. Originally, cyclophilins were identified as the intracellular receptors of the immunosuppressive drug cyclosporin A. Cyclophilins play a major role in protein folding and they act as molecular chaperones. Furthermore, they have Mg²⁺ - and Ca²⁺-dependent nuclease activity and are discussed to be involved in apoptosis. Among all cyclophilin family members, cyclophilin A (PPIA) was first reported to be up-regulated in various malignant diseases. Overexpression of PPIA was reported to protect cancer cells against cellular stress induced by cisplatinum and hypoxia though contributing to platinum resistance. Whether combining cyclosporine A with cisplatinum can help to overcome PPIA induced chemoresistance in patients with malignant glioma is currently under investigation. In our study, PPIC positive CTC were more likely in follow-up samples of the platinum resistant patients, but not in patients with older age or FIGO stage IV, who were generally more often CTC positive. In view of this observation and of the close structural and functional similarity of members of the cyclophilin family, we speculate that PPIC positive CTC represent a subpopulation of circulating tumor cells which "survived" chemotherapy and have a more aggressive potential.

This hypothesis is supported by the fact that in our study PPIC positive patients had a poor prognosis, which resulted from PPIC mediated chemoresistence, suggesting that PPIC might be a promising target for the therapy of chemoresistant EOC.

It is remarkable, that PPIC is not only the most prominent CTC marker in the present EOC study, but was the most prominent marker also in a differently designed previous study by our group that in addition to EOC patients investigated patients with breast, endometrial and cervical cancer. Even though the experimental approach regarding microarray analysis (cell lines vs. tumor tissue), cDNA synthesis (amplified vs. non-amplified RNA samples), and PCR platform (microfluidic cards vs. microplates) was quite different in that previous study, PPIC was part of a gene panel including CCNE2, MAL2, EMP2, HJURP, and SLC6A8. Out of this panel in the present study only MAL2 was differentially expressed in tumor tissue, MAL2 was excluded as a promising marker for CTC detection because of detectable background gene expression in the control blood samples. However, neither the presence of CTC in baseline nor in follow-up samples was related with distant metastasis. Furthermore there was no correlation between PPIC positive CTC and PPIC gene expression in the matched tumor tissue. There are two possible explanations. First, false negative CTC results caused either by loss of CTC during density gradient centrifugation, or by expression below the defined standard deviations above the mean control values. Second, false negative PPIC gene expression in the tumor tissue samples, caused either by the less sensitive microarray technology that was used to detect PPIC gene expression in tumor tissue, compared to the more quantitative RT-qPCR used to detect CTC, or by possible heterogeneity of PPIC gene expression in tumor tissues.

To our knowledge, this is the first study demonstrating the impact of baseline and follow-up CTC on both DFS and OS in EOC. Furthermore, to our knowledge, this is the first report to demonstrate that the prognostic information derived from CTC positivity is largely independent to other classical clinicopathological parameters. Applying Cox regression analysis we could demonstrate that PPIC positive CTC detected before primary treatment are an independent poor prognostic factor for both DFS and OS. Interestingly, the absence of PPIC positive CTC did not confer a better prognosis to the EOC patients in our study. After completion of adjuvant chemotherapy, PPIC positive CTC independently predicted poor DFS. For OS we could only demonstrate a trend, which might become significant with longer follow-up and an increasing number of events. To summarize, we could demonstrate that PPIC positive CTC have a stronger direct impact on poor survival.

As a conclusion, we could identify novel and clinically relevant markers for sensitive detection of CTC in EOC patients. The detection of CTCs over-expressing PPIC indicated particularly aggressive EOC, since the presence of PPIC positive CTC both, before primary treatment and six months after completion of adjuvant chemotherapy predicted an adverse outcome. Our novel gene markers allow for further characterization of particularly aggressive EOC by CTC diagnostics based on PPIC gene over-expression, potentially contributing to the development of new targeted anti PPIC therapies and thus, more personalized treatment strategies for EOC patients.

**Table 7: Marker panel for the RT-qPCR based detection of CTC. Eleven genes were identified as markers for circulating tumor cells in EOC patients. Additionally, the EpCAM gene expression was analyzed in the same samples with RT-qPCR. CTC positivity was defined by over-expression of at least one of the 11 gene markers. The numbers of positive EOC blood samples taken at baseline (before primary treatment) and during follow-up (six months after chemotherapy) are given.**

| Gene symbol | Gene name | baseline | follow-up |
|---|---|---|---|
| PPIC | Cyclophilin C | 34 (17.0%) | 13 (14.0%) |
| GPX8 | Probable glutathione peroxidase 8 | 11 (5.5%) | 1 (1.1%) |
| CDH3 | Cadherin-3 | 8 (4.0%) | 2 (2.2%) |
| TUSC3 | Tumor suppressor candidate 3 | 7 (3.5%) | 1 (1.1%) |
| COL3A1 | Collagen alpha-1(III) chain | 3 (1.5%) | 1 (1.1%) |
| LAMB1 | Laminin subunit beta-1 | 3 (1.5%) | 1 (1.1%) |
| MAM | Mammaglobin A | 2 (1.0%) | 0 |
| ESRP2 | Epithelial splicing regulatory protein 2 | 2 (1.0%) | 1 (1.1%) |
| AGR2 | Anterior gradient protein 2 homolog | 1 (0.5%) | 1(1.1%) |
| BAIAP2L1 | Brain-specific angiogenesis inhibitor 1-associated protein 2-like protein 1 | 0 | 0 |
| TFF1 | Trefoil factor 1 | 0 | 0 |
| EPCAM | Epithelial cell adhesion molecule | 4 (2.0%) | 1 (1.1%) |

**Table 8: Presence of CTC and correlation to clinicopathologic characteristics of the patients**

| | **baseline** | | | | **follow-up** | | | |
|---|---|---|---|---|---|---|---|---|
| | **N** | **CTC+** | **CTC-** | **p** | **N** | **CTC+** | **CTC-** | **p** |
| **Total cases** | 200 | 49 (24%) | 151 (76%) | | 93 | 19 (20%) | 74 (80%) | |
| **Mean age** (yrs) | | 58.9 (±12.7) | 58.2 (±11.5) | n.s. | | 1 62.6 (±12.1) | 55.2 (±11.6) | 0.016 |
| **FIGO stage** | | | | n.s. | | | | 0.035 |
| II/III | 164 | 38 (23%) | 126 (77%) | | 79 | 13 (17%) | 66 (83%) | |
| IV | 36 | 11 (31%) | 25 (69%) | | 14 | 6 (43%) | 8 (57%) | |
| **Grade** | | | | n.s. | | | | n.s. |
| 2 | 58 | 12 (21%) | 46 (79%) | | 24 | 2 (8%) | 22 (92%) | |
| 3 | 144 | 37 (26%) | 104 (74%) | | 69 | 17 (25%) | 52 (75%) | |
| **Lymph node involvement** | | | | n.s. | | | | n.s. |
| | 112 | 30 (27%) | 82 (73%) | | 46 | 8 (17%) | 38 (83%) | |
| yes | 45 | 6 (13%) | 39 (87%) | | 19 | 3 (16%) | 16 (84%) | |
| no | | | | | | | | |
| **Response to therapy** | | | | n.s. | | | | 0.035 |
| yes | 155 | 36 (23%) | 119 (77%) | | 79 | 13 (16%) | 66 (84%) | |
| no | 44 | 13 (30%) | 31 (70%) | | 14 | 6 (43%) | 8 (57%) | |
| **Residual disease** | | | | 0.013 | | | | n.s |
| yes | 66 | 23 (35%) | 43 (65%) | | 26 | 6 (23%) | 20 (77%) | |
| no | 133 | 25 (19%) | 108 (81%) | | 66 | 13 (20%) | 53 (80%) | |
| **Peritoneal carcinomatosis** | | | | n.s. | | | | n.s. |
| | 135 | 36 (27%) | 99 (73%) | | 66 | 16 (24%) | 50 (76%) | |
| yes | 64 | 12 (19%) | 52 (81%) | | 26 | 3 (12%) | 23 (88%) | |
| no | | | | | | | | |
| **Ascites** | | | | 0.021 | | | | n.s. |
| yes | 146 | 42 (29%) | 104 (71%) | | 72 | 15 (21%) | 57 (79%) | |
| no | 54 | 7 (13%) | 47 (87%) | | 21 | 4 (19%) | 17 (81%) | |

**Table 9: Cox's proportional hazard regression models for disease free survival**

| | **baseline** | | | | | | | | **follow-up** | | | | | | | |
|---|---|---|---|---|---|---|---|---|---|---|---|---|---|---|---|---|
| | **univariate** | | | | **multiple** | | | | **univariate** | | | | **multiple** | | | |
| | **HR** | **95% CI** | | **p** | **HR** | **95% CI** | | **p** | **HR** | **95% CI** | | **p** | **HR** | **95% CI** | | **p** |
| **Age** | 1.017 | 1.002 | 1.033 | 0.031 | * | | | | 1.007 | 0.985 | 1.029 | 0.554 | * | | | |
| **FIGO stage ^{‡}** | 1.962 | 1.293 | 2.979 | 0.002 | 1.604 | 1.038 | 2.479 | 0.034 | 2.919 | 1.530 | 5.568 | 0.001 | 2.418 | 1.225 | 4.774 | 0.011 |
| **Residual disease^{†}** | 2.172 | 1.518 | 3.108 | <0.001 | 1.531 | 1.048 | 2.238 | 0.028 | 2.026 | 1.189 | 3.453 | 0.009 | * | | | |
| **Peritoneal carcinomatosis** ^{†} | 2.479 | 1.633 | 3.763 | <0.001 | 2.235 | 1.450 | 3.447 | <0.001 | 3.167 | 1.694 | 5.923 | <0.001 | 2.844 | 1.495 | 5.410 | 0.001 |
| **Ascites ^{†}** | 1.878 | 1.219 | 2.892 | 0.004 | * | | | | 2.912 | 1.431 | 5.924 | 0.003 | 2.809 | 1.319 | 5.983 | 0.007 |
| **PPIC status ^{†}** | 1.765 | 1.134 | 2.746 | 0.012 | 1.643 | 1.046 | 2.580 | 0.031 | 2.756 | 1.455 | 5.220 | 0.002 | 3.415 | 1.749 | 6.668 | <0.001 |

| | | | | | | | | | | | | | | | | |
|---|---|---|---|---|---|---|---|---|---|---|---|---|---|---|---|---|
| Cl, confidence interval; HR, hazard ratio; * not significant in the final multiple regression model; † positive versus negative; ‡ IV versus II and III | | | | | | | | | | | | | | | | |

**Table 10: Cox's proportional hazard regression models for overall survival**

| | **baseline** | | | | | | | | **follow-up** | | | | | | | |
|---|---|---|---|---|---|---|---|---|---|---|---|---|---|---|---|---|
| | **univariate** | | | | **multiple** | | | | **univariate** | | | | **multiple** | | | |
| | **HR** | **95% CI** | | **p** | **HR** | **95% CI** | | **p** | **HR** | **95% CI** | | **p** | **HR** | **95% CI** | | **p** |
| **Age** | 1.033 | 1.007 | 1.060 | 0.012 | 1.029 | 1.004 | 1.056 | 0.024 | 1.046 | 1.009 | 1.084 | 0.014 | 1.041 | 1.006 | 1.077 | 0.020 |
| **FIGO stage ^{‡}** | 1.869 | 0.972 | 3.593 | 0.061 | * | | | | 1.067 | 0.315 | 3.617 | 0.917 | * | | | |
| **Residual disease^{†}** | 1.995 | 1.148 | 3.468 | 0.014 | * | | | | 1.749 | 0.706 | 4.336 | 0.227 | * | | | |
| **Peritoneal carcinomatosis ^{†}** | 3.134 | 1.471 | 6.680 | 0.003 | 2.770 | 1.294 | 5.928 | 0.009 | 6.075 | 1.414 | 26.093 | 0.015 | 5.878 | 1.367 | 25.278 | 0.017 |
| | 2.158 | 1.014 | 4.594 | 0.046 | * | | | | 3.882 | 0.903 | 16.683 | 0.068 | * | | | |
| **PPIC status ^{†}** | 2.537 | 1.385 | 4.650 | 0.003 | 2.229 | 1.213 | 4.097 | 0.010 | 2.927 | 1.050 | 8.155 | 0.040 | * | | | |

| | | | | | | | | | | | | | | | | |
|---|---|---|---|---|---|---|---|---|---|---|---|---|---|---|---|---|
| Cl, confidence interval; HR, hazard ratio; * not significant in the final multiply regression model; † positive versus negative; ‡ IV versus II and III | | | | | | | | | | | | | | | | |

### REFERENCES

1. Brandt B, Griwatz C: Two-layer buoyant density centrifugation gradient for enrichment of prostate-derived cells and cell clusters from peripheral blood. Clin Chem 1996, 42(11):1881-1882.
2. Mikhitarian K, Martin RH, Ruppel MB, Gillanders WE, Hoda R, Schutte del H, Callahan K, Mitas M, Cole DJ: Detection of mammaglobin mRNA in peripheral blood is associated with high grade breast cancer: interim results of a prospective cohort study. BMC Cancer 2008, 8:55.
3. Lee J, Kim SS: Current implications of cyclophilins in human cancers. J Exp Clin Cancer Res, 29:97.
4. Mi Z, Oliver T, Guo H, Gao C, Kuo PC: Thrombin-cleaved COOH(-) terminal osteopontin peptide binds with cyclophilin C to CD147 in murine breast cancer cells. Cancer Res 2007, 67(9):4088-4097.
5. Aberger F, Weidinger G, Grunz H, Richter K: Anterior specification of embryonic ectoderm: the role of the Xenopus cement gland-specific gene XAG-2. Mech Dev 1998, 72(1-2):115-130.
6. Smirnov DA, Foulk BW, Doyle GV, Connelly MC, Terstappen LW, O'Hara SM: Global gene expression profiling of circulating endothelial cells in patients with metastatic carcinomas. Cancer Res 2006, 66(6):2918-2922.
7. Watson MA, Fleming TP: Mammaglobin, a mammary-specific member of the uteroglobin gene family, is overexpressed in human breast cancer. Cancer Res 1996, 56(4):860-865.
8. Klug J, Beier HM, Bernard A, Chilton BS, Fleming TP, Lehrer RI, Miele L, Pattabiraman N, Singh G: Uteroglobin/Clara cell 10-kDa family of proteins: nomenclature committee report. Ann N Y Acad Sci 2000, 923:348-354.
9. Brown NM, Stenzel TT, Friedman PN, Henslee J, Huper G, Marks JR: Evaluation of expression based markers for the detection of breast cancer cells. Breast Cancer Res Treat 2006, 97(1):41-47.
10. Grunewald K, Haun M, Urbanek M, Fiegl M, Muller-Holzner E, Gunsilius E, Dunser M, Marth C, Gastl G: Mammaglobin gene expression: a superior marker of breast cancer cells in peripheral blood in comparison to epidermal-growth-factor receptor and cytokeratin-19.-Lab Invest 2000, 80(7):1071-1077.
11. Zafrakas M, Petschke B, Donner A, Fritzsche F, Kristiansen G, Knuchel R, Dahl E: Expression analysis of mammaglobin A (SCGB2A2) and lipophilin B (SCGB1 D2) in more than 300 human tumors and matching normal tissues reveals their co-expression in gynecologic malignancies. BMC Cancer 2006, 6:88.
12. Abdou AG, Aiad HA, Sultan SM: pS2 (TFF1) expression in prostate carcinoma: correlation with steroid receptor status. Apmis 2008, 116(11):961-971.
13. Warzecha CC, Sato TK, Nabet B, Hogenesch JB, Carstens RP: ESRP1 and ESRP2 are epithelial cell-type-specific regulators of FGFR2 splicing. Mol Cell 2009, 33(5):591-601.
14. Aktas B, Kasimir-Bauer S, Heubner M, Kimmig R, Wimberger P. Molecular Profiling and Prognostic Relevance of Circulating Tumor Cells in the Blood of Ovarian Cancer Patients at Primary Diagnosis and After Platinum-Based Chemotherapy. Int J Gynecol Cancer. 2011.
15. Poveda A, Kaye SB, McCormack R, Wang S, Parekh T, Ricci D, et al. Circulating tumor cells predict progression free survival and overall survival in patients with relapsed/recurrent advanced ovarian cancer. Gynecol Oncol. 2011.
16. Marth C, Kisic J, Kaern J, Trope C, Fodstad O. Circulating tumor cells in the peripheral blood and bone marrow of patients with ovarian carcinoma do not predict prognosis. Cancer. 2002;94:707-12.

## Claims

1. A method of determining ovarian cancer disease in a subject, which comprises
- measuring the PPIC expression of cells in a sample of peripheral blood of the subject, and
- comparing to a reference value,
the PPIC overexpression being indicative of a ovarian cancer disease and/or disease progression.

2. Method according to claim 1, which employs a marker panel comprising PPIC and at least one further marker.

3. Method according to claim 1 or 2, which further comprises
- additionally determining expression of CDH3 in said sample,
wherein the CDH3 expression is indicative of an improved prognosis.

4. Method according to any of claims 1 to 3, wherein at least one further marker selected from the group consisting of GPX8, TUSC3, AGR2, COL3A1, LAMB1, MAM, TFF1, BAIAP2L1, ESRP2 and EpCAM is determined.

5. Method according to any of claims 2 to 4, wherein said marker panel does not comprise CCNE2 or MAL2.

6. Method according to any of claims 1 to 5, wherein the expression of PPIC and/or at least one further marker is quantitatively determined.

7. Method according to any of claims 1 to 6, wherein the nucleic acid and/or protein expression is determined, optionally employing amplification methods, among them nucleic acid amplification methods, RT-qPCR, microarrays, immunoassays, such as ELISA, EIA, RIA, western blot, protein arrays, immunocytochemistry or immunohistochemistry methods.

8. Method according to any of claims 1 to 7, wherein said sample is obtained from a blood fraction containing mononuclear cells, such as a PBMC fraction, preferably wherein said sample is enriched in circulating tumor cells.

9. Method according to any of claims 1 to 8, wherein the reference value is determined in a sample of a healthy subject.

10. Method according to any of claims 1 to 9, wherein said subject is suffering from early stage cancer.

11. Method according to any of claims 1 to 10, wherein said subject is undergoing or has received chemotherapy, and the risk of disease progression is determined.

12. Method according to any of claims 1 to 11, wherein the subject is suffering from minimal residual disease.

13. Method according to any of claims 1 to 12, wherein the PPIC overexpression is indicative of an increased metastatic potential associated with a shortened survival time.

14. Use of a method according to any one of claims 1 to 13, for diagnosing or aiding in the diagnosis of ovarian cancer.

15. Use of a method according to any one of claims 1 to 14, for the determination of the metastatic potential in an ovarian cancer patient at risk of disease progression and/or for monitoring the disease progression of ovarian cancer in a patient.

## Patentansprüche

1. Verfahren zum Bestimmen von Eierstockkrebs bei einer Person, wobei das Verfahren umfasst:
- Messen der PPIC-Expression von Zellen in einer Probe aus peripherem Blut der Person, und
- Vergleichen mit einem Referenzwert,
wobei die PPIC-Überexpression einen Hinweis auf eine Erkrankung an Eierstockkrebs und/oder auf einen progressiven Krankheitsverlauf liefert.

2. Verfahren nach Anspruch 1, das ein Marker-Panel anwendet, das einen PPIC-Marker und mindestens einen weiteren Marker umfasst.

3. Verfahren nach Anspruch 1 oder 2, das ferner umfasst:
- zusätzliches Bestimmen einer Expression von CDH3 in der Probe,
wobei die CDH3-Expression einen Hinweis auf eine verbesserte Prognose liefert

4. Verfahren nach einem der Ansprüche 1 bis 3, wobei mindestens ein weiterer Marker, der ausgewählt ist aus der Gruppe, die aus GPX8, TUSC3, AGR2, COL3A1, LAMB1, MAM, TFF1, BAIAP2L1, ESRP2 und EpCAM besteht, bestimmt wird.

5. Verfahren nach einem der Ansprüche 2 bis 4, wobei das Marker-Panel nicht CCNE2 oder MAL2 umfasst.

6. Verfahren nach einem der Ansprüche 1 bis 5, wobei die Expression von PPIC und/oder von mindestens einem weiteren Marker quantitativ bestimmt wird.

7. Verfahren nach einem der Ansprüche 1 bis 6, wobei die Nukleinsäure und/oder die Proteinexpression bestimmt wird, indem wahlweise Amplifikationsverfahren angewandt werden, darunter die Amplifikationsverfahren für Nukleinsäuren, RT-qPCR, Microarrays, Immuntests, wie zum Beispiel ELISA, EIA, RIA, Western-Blot, Proteinarrays, Immunzytochemie oder immunhistochemische Verfahren.

8. Verfahren nach einem der Ansprüche 1 bis 7, wobei die Probe aus einer Blutfraktion erhalten wird, die mononukleare Zellen wie etwa eine PBMC-Fraktion enthält, wobei die Probe vorzugsweise an zirkulierenden Tumorzellen angereichert ist.

9. Verfahren nach einem der Ansprüche 1 bis 8, wobei der Referenzwert in einer Probe einer gesunden Person bestimmt wird.

10. Verfahren nach einem der Ansprüche 1 bis 9, wobei die Person an Krebs in einem frühen Stadium leidet.

11. Verfahren nach einem der Ansprüche 1 bis 10, wobei die Person eine Chemotherapie durchläuft oder erhalten hat, und wobei das Risiko eines progressiven Krankheitsverlaufs bestimmt wird.

12. Verfahren nach einem der Ansprüche 1 bis 11, wobei die Person an einer minimalen Resterkrankung leidet.

13. Verfahren nach einem der Ansprüche 1 bis 12, wobei die PPIC-Überexpression einen Hinweis auf ein erhöhtes Metastasenpotential liefert, das mit einer verkürzten Überlebenszeit verbunden ist.

14. Verwendung eines Verfahrens nach einem der Ansprüche 1 bis 13, um einen Eierstockkrebs zu diagnostizieren oder um bei der Diagnose des Eierstockkrebses zu helfen.

15. Verwendung eines Verfahrens nach einem der Ansprüche 1 bis 14 für die Bestimmung des Metastasenpotentials bei einem Patienten mit Eierstockkrebs mit dem Risiko eines progressiven Krankheitsverlaufs und/oder zum Überwachen des progressiven Krankheitsverlaufs eines Eierstockkrebses bei einem Patienten.

## Revendications

1. Procédé de détermination d'une pathologie de type cancer de l'ovaire chez un sujet, qui comprend :
- la mesure de l'expression de PPIC dans les cellules d'un échantillon de sang périphérique du sujet, et
- la comparaison à une valeur de référence,
la surexpression de PPIC indiquant une pathologie de type cancer de l'ovaire et/ou la progression de la maladie.

2. Procédé selon la revendication 1, qui utilise un panel de marqueurs comprenant le PPIC et au moins un autre marqueur.

3. Procédé selon la revendication 1 ou 2, qui comprend en outre
- la détermination supplémentaire de l'expression de CDH3 dans ledit échantillon,
dans lequel l'expression de CDH3 indique un meilleur pronostic.

4. Procédé selon l'une quelconque des revendications 1 à 3, dans lequel au moins un autre marqueur sélectionné dans le groupe constitué de GPX8, TUSC3, AGR2, COL3A1, LAMB1, MAM, TFF1, BAIAP2L1, ESRP2 et EpCAM est déterminé.

5. Procédé selon l'une quelconque des revendications 2 à 4, dans lequel ledit panel de marqueurs ne comprend pas CCNE2 ou MAL2.

6. Procédé selon l'une quelconque des revendications 1 à 5, dans lequel l'expression de PPIC et/ou d'au moins un autre marqueur est quantitativement déterminée.

7. Procédé selon l'une quelconque des revendications 1 à 6, dans lequel l'expression de l'acide nucléique et/ou de la protéine est déterminée, facultativement en utilisant des procédés d'amplification, parmi lesquels des procédés d'amplification d'acides nucléiques, la RT-qPCR, des micropuces à ADN, des dosages immunologiques, comme un ELISA, un EIA, un RIA, un transfert de type Western, des réseaux de protéines, des procédés d'immunocytochimie ou d'immunohistochimie.

8. Procédé selon l'une quelconque des revendications 1 à 7, dans lequel ledit échantillon est obtenu d'une fraction de sang contenant des cellules mononucléaires, par exemple une fraction de PBMC, de préférence dans lequel ledit échantillon est enrichi en cellules tumorales circulantes.

9. Procédé selon l'une quelconque des revendications 1 à 8, dans lequel la valeur de référence est déterminée dans un échantillon d'un sujet sain.

10. Procédé selon l'une quelconque des revendications 1 à 9, dans lequel ledit sujet sain souffre d'un cancer de stade précoce.

11. Procédé selon l'une quelconque des revendications 1 à 10, dans lequel ledit sujet subit ou a reçu une chimiothérapie, et le risque de progression de la maladie est déterminé.

12. Procédé selon l'une quelconque des revendications 1 à 11, dans lequel le sujet souffre d'une maladie résiduelle minime.

13. Procédé selon l'une quelconque des revendications 1 à 12, dans lequel la surexpression de PPIC indique un potentiel métastatique accru associé à une durée de survie réduite.

14. Utilisation d'un procédé selon l'une quelconque des revendications 1 à 13, pour le diagnostic ou l'aide au diagnostic du cancer de l'ovaire.

15. Utilisation d'un procédé selon l'une quelconque des revendications 1 à 14, pour la détermination du potentiel métastatique chez une patiente atteinte du cancer de l'ovaire à risque de progression de la maladie et/ou pour la surveillance de la progression de la maladie du cancer de l'ovaire chez une patiente.


### SEQUENCE LISTING

<110> Zeillinger, Robert
<120> Novel tumor marker determination
<130> ZE004P
<160> 2
<170> PatentIn version 3.5
<210> 1
   <211> 212
   <212> PRT
   <213> Homo sapiens
<400> 1
   Met Gly Pro Gly Pro Arg Leu Leu Leu Pro Leu Val Leu Cys Val Gly
   1 5 10 15
   Leu Gly Ala Leu Val Phe Ser Ser Gly Ala Glu Gly Phe Arg Lys Arg
   20 25 30
   Gly Pro Ser Val Thr Ala Lys Val Phe Phe Asp Val Arg Ile Gly Asp
   35 40 45
   Lys Asp Val Gly Arg Ile Val Ile Gly Leu Phe Gly Lys Val Val Pro
   50 55 60
   Lys Thr Val Glu Asn Phe Val Ala Leu Ala Thr Gly Glu Lys Gly Tyr
   65 70 75 80
   Gly Tyr Lys Gly Ser Lys Phe His Arg Val Ile Lys Asp Phe Met Ile
   85 90 95
   Gln Gly Gly Asp Ile Thr Thr Gly Asp Gly Thr Gly Gly Val Ser Ile
   100 105 110
   Tyr Gly Glu Thr Phe Pro Asp Glu Asn Phe Lys Leu Lys His Tyr Gly
   115 120 125
   Ile Gly Trp Val Ser Met Ala Asn Ala Gly Pro Asp Thr Asn Gly Ser
   130 135 140
   Gln Phe Phe Ile Thr Leu Thr Lys Pro Thr Trp Leu Asp Gly Lys His
   145 150 155 160
   Val Val Phe Gly Lys Val Ile Asp Gly Met Thr Val Val His Ser Ile
   165 170 175
   Glu Leu Gln Ala Thr Asp Gly His Asp Arg Pro Leu Thr Asn Cys Ser
   180 185 190
   Ile Ile Asn Ser Gly Lys Ile Asp Val Lys Thr Pro Phe Val Val Glu
   195 200 205
   Ile Ala Asp Trp
   210
<210> 2
   <211> 883
   <212> DNA
   <213> Homo sapiens
<400> 2
   caattattcc aaatctattc cactctcttc aagcctgtac cccacctgcc ctttctcagc     60
   aggctgctcc tctcacttct aggagtcccg tcagctgtcc cagagcctgt gtggcgcccg    120
   tgccggtagc gcccgtgccg gtagcgccgc tgccaccgct caccatgggc ccgggtcctc    180
   ggctgctgct acctctcgtg ctttgcgtgg ggctcggcgc acttgtgttt tcttcggggg    240
   ccgagggctt ccgcaagcga ggcccctcgg tgacggccaa ggtcttcttt gatgtgagga    300
   ttggagacaa agatgttggc agaattgtga ttggcctctt tggaaaagtt gtgcccaaga    360
   cagtggaaaa ttttgttgct ctagcaacag gagagaaagg atatggatat aaaggaagca    420
   agtttcatcg tgtcatcaag gatttcatga ttcaaggagg tgacatcacc actggagatg    480
   gcactggggg tgtgagcatc tatggtgaga catttccaga tgagaacttc aagctgaagc    540
   actatggcat tgggtgggtc agcatggcca acgctgggcc tgacaccaat ggctctcagt    600
   tctttatcac cttgaccaag cccacctggt tggacggcaa acatgtggtg tttggaaaag    660
   tcattgatgg gatgacagtg gtgcactcca tagagctcca agcaactgat gggcatgacc    720
   gtccactcac caactgctcg atcatcaaca gtggcaagat agacgtgaaa acgccttttg    780
   tggttgagat cgctgattgg tgacacaact ggcagaaaac aaggatatgc tttggcaggg    840
   gtgtgtgtgt gtgtgtgtgt gtgtgtgtgt tgtgttgtct ttc                     883